(19) Europäisches Patentamt
European Patent Office
Office européen des brevets

(11) EP 3 024 442 B1

(12) EUROPEAN PATENT SPECIFICATION

(45) Date of publication and mention of the grant of the patent:
**16.01.2019 Bulletin 2019/03**

(21) Application number: **14741876.8**

(22) Date of filing: **22.07.2014**

(51) Int Cl.:
***A61K 9/16*** (2006.01) ***A61K 9/20*** (2006.01)
***A61K 31/70*** (2006.01)

(86) International application number:
**PCT/EP2014/065661**

(87) International publication number:
**WO 2015/011113 (29.01.2015 Gazette 2015/04)**

(54) **FORMULATIONS CONTAINING AMORPHOUS DAPAGLIFLOZIN**

ZUSAMMENSETZUNGEN MIT AMORPHEM DAPAGLIFLOZIN

COMPOSITIONS COMPRENANT DAPAGLIFLOZIN AMORPHE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.07.2013 EP 13177508**

(43) Date of publication of application:
**01.06.2016 Bulletin 2016/22**

(73) Proprietor: **Sandoz AG**
**4056 Basel (CH)**

(72) Inventors:
- **STARIC, Rok**
  **1526 Ljubljana (SI)**
- **BERGLEZ, Sandra**
  **1526 Ljubljana (SI)**
- **GRMAS, Jernej**
  **1526 Ljubljana (SI)**
- **STANIC LJUBIN, Tijana**
  **1526 Ljubljana (SI)**
- **GRAHEK, Rok**
  **1526 Ljubljana (SI)**
- **PETERNEL, Luka**
  **1526 Ljubljana (SI)**

(74) Representative: **Prüfer & Partner mbB**
**Patentanwälte · Rechtsanwälte**
**Sohnckestraße 12**
**81479 München (DE)**

(56) References cited:
**WO-A1-2008/116179 WO-A1-2012/163546**
**WO-A2-2010/045656 WO-A2-2011/060290**
**WO-A2-2012/031124**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 024 442 B1

## Description

Field of the invention

The present invention belongs to the field of pharmaceutical industry and relates to pharmaceutical compositions containing amorphous dapagliflozin, in particular in the form of solid dispersions and adsorbates, and a process for preparing the same. Furthermore, the invention relates to the use of said pharmaceutical compositions for use in the treatment of diseases related to hypoglycemia.

Description of the background art

Diabetes is a group of metabolic diseases in which a person has high blood sugar, because the pancreas does not produce enough insulin, and/or because cells do not respond to the insulin that is produced. This high blood sugar produces the classical symptoms of polyuria (frequent urination), polydipsia (increased thirst) and polyphagia (increased hunger). Currently, there exist three types of diabetes, type 1 diabetes (type 1), type 2 diabetes (type 2), and gestational diabetes. Gestational diabetes occurs in pregnant women who develop high blood glucose level. Type 1 diabetes results from the body's failure to produce insulin, and thus the injection of insulin is necessary. Finally, with type 2 diabetes, the body either resists the effects of insulin, or does not produce enough insulin to maintain a normal glucose level. Of these three types of diabetes, type 2 diabetes is the most common form of diabetes, with worldwide more than 171 million people suffering from it.

It is known that the subtype 2 of the sodium-glucose transport proteins (SGLT2) are predominantly expressed in the renal proximal tubules. Further, it is assumed that these proteins are the major transporter that are responsible for at least 90% of the glucose reabsorption in the kidney.

The active pharmaceutical ingredient (API) dapagliflozin is reported to inhibit SGLT2. Bristol-Myers Squibb (BMS) and AstraZeneca have developed and launched dapagliflozin propanediol (also designated as Forxiga or BMS-512148), an orally active sodium glucose cotransporter type 2 (SGLT-2) inhibitor. The product is indicated in Europe for the once-daily treatment of type 2 diabetes, as an adjunct to diet and exercise in combination with other glucose-lowering medicinal products, including insulin, or as a monotherapy in metformin intolerant patients. Development is also ongoing for other indications (such as type 1 diabetes and other conditions resulting in hyperglycemia).

The compound was also designated as "(2S,3R,4R,5S,6R)-2-[4-chloro-3-[(4-ethoxyphenyl)methyl]phenyl]-6-(hydroxymethyl)oxane-3,4,5-triole", or as "(2S,3R,4R,5S,6R)-2-[4-chloro-3-(4-ethoxybenzyl)phenyl]-6-(hydroxymethyl)tetrahydro-2H-pyran-3,4,5-triole", depicted below:

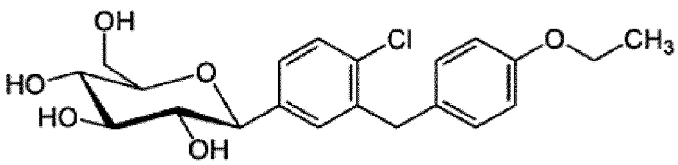

In the past, various attempts have been made to synthesize dapagliflozin and to prepare pharmaceutical formulations thereof. For instance, the synthesis of dapagliflozin is described in WO 03/099836 A1.

WO 2008/002824 concerns crystalline forms and solvates of (1S)-1,5-anhydro-1-C-(3-((phenyl)methyl)phenyl)-D-glucitol derivatives and their complexes with amino acids. In particular, it discloses free acid polymorphic crystal structures of dapagliflozin, for example in the form of a propylene glycol hydrate. WO 2008/116179 refers to pharmaceutical formulations which include crystalline dapagliflozin propylene glycol hydrate. WO 2012/163546 discloses pharmaceutical compositions comprising dapagliflozin and cyclodextrin, which compositions are in the form of inclusion bodies. Crystalline dapagliflozin hydrate and a process for obtaining the same is disclosed in WO 2013/079501.

Bristol-Myers Squibb (BMS) and AstraZeneca are also developing a fixed dose combination of dapagliflozin and metformin for type 2 diabetes. For instance, WO 2011/060256 discloses bilayered tablets containing dapagliflozin and metformin. WO 2011/060290 discloses a process of preparing an immediate release formulation comprising a combination of dapagliflozin and metformin.

WO 03/000238 relates to adsorbates of low-solubility drugs that have been formed by rapidly removing the solvent. WO 2009/080698 refers to a combination of poorly soluble API with pharmaceutically acceptable solid supports. US 2012/0088774 (also published as WO 2010/115886) refers to an active pharmaceutical ingredient (API) being practically insoluble in water associated with a particulate and/or porous substrate where the API is adsorbed on the solid support.

Although several solid forms of dapagliflozin are known in the art, finding a good or even the optimal form with

regard to bioavailability, inter-patient variability, and safety remains a considerable challenge, in particular when the compound forms many polymorphic forms. Not all solid forms of dapagliflozin are equally suitable with regard to stability, flow properties, compressibility, dissolution rate. For instance, amorphous forms can be thought of as liquids that have been solidified by the removal of thermal energy or a solvent, in a manner that circumvents crystallization. The amorphous form can have different solubility, stability, and mechanical behaviour that can be exploited by pharmaceutical scientists. However, although amorphous forms are sometimes better soluble than crystalline forms, they are often not the preferred form because of water activity and/or stability reasons.

It therefore not only remains a considerable challenge finding a good or even the optimal form with regard to bioavailability, inter-patient variability, and safety, but also providing a good or even the optimal formulation. Hence, despite the above-described methods and preparations of formulations containing dapagliflozin, there is a need and thus an object of the present invention for an improved composition containing said pharmaceutically active ingredient (API), in particular with regard to an improved dissolution profile, improved content uniformity, and/or improved processability. Further, there is a need for a pharmaceutical composition comprising said API exhibiting a satisfying (storage) stability while at the same time the method of preparing said composition is improved e.g. in terms of time or effort, for instance with respect to (mechanical) equipment needed for the preparation, or educts needed. Thus, there is also a need for an improved process for the preparation of said formulations.

An object of the present invention was to find a pharmaceutical composition comprising dapagliflozin which exhibits improved chemical stability upon storage. It was a further object of the present invention to find a pharmaceutical composition comprising dapagliflozin, which composition has defined solid state characteristics for the API, in particular avoiding undesired conversion to any other solid state form taking place during the formulation process. It was a further object of the present invention to find a pharmaceutical composition comprising dapagliflozin, which composition has improved disintegration characteristics, in particular after prolonged storage at conditions which are typical for tropical countries. It was a further object of the present invention to find a pharmaceutical composition comprising dapagliflozin, which composition is suitable for storage in a packaging material with a high moisture vapour transmission rate.

These objects as well as others, which will become apparent from the following description of the present invention, are attained by the subject-matter of the independent claims. Some of the preferred embodiments of the present invention are defined by the subject matter of the dependent claims.

## Summary of the invention

The present invention provides the following aspects, subject-matters and preferred embodiments, which respectively taken alone or in combination, contribute to solving the object of the present invention:
The present invention refers to the following items:

(1) Amorphous solid dispersion of at least one suitable polymer and dapagliflozin ((2S,3R,4R,5S,6R)-2-[4-chloro-3-(4-ethoxybenzyl)phenyl]-6-(hydroxymethyl)-tetrahydro-2H-pyran-3,4,5-triol) of formula 1

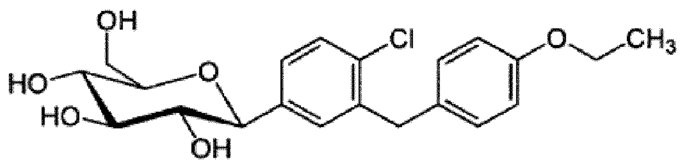

formula 1.

According to the invention, the term "suitable polymer" means a polymer which is suitable for forming an amorphous solid dispersion with dapagliflozin.
In a preferred embodiment, the solid dispersion is a solid solution.

(2) The solid dispersion according to item 1, wherein the solid dispersion is substantially homogeneous.

(3) The solid dispersion according to item 1 or 2, wherein the at least one polymer is selected from the group consisting of polyvinyl pyrrolidone (PVP), polyvinyl alcohol (PVA), polyacrylic acid (PAA), poly(ethylene glycol) (PEG), poly(ethylene oxide) (PEO), hydroxypropyl cellulose (HPC), hydroxypropyl methyl cellulose (HPMC), copovidone, hypromellose acetate succinate (AQOAT), polyacrylates and mixtures thereof.
In a preferred embodiment, the at least one polymer is preferably selected from the group consisting of PVP, PVA, HPC and HPMC.
In an even more preferred embodiment, the at least one polymer is selected grom the group consisting of polyvinyl pyrrolidone (PVP) and polyvinyl alcohol (PVA).

This embodiment thus concerns an amorphous solid dispersion of dapagliflozin ((2S,3R,4R,5S,6R)-2-[4-chloro-3-(4-ethoxybenzyl)phenyl]-6-(hydroxymethyl)-tetrahydro-2H-pyran-3,4,5-triol) of formula 1

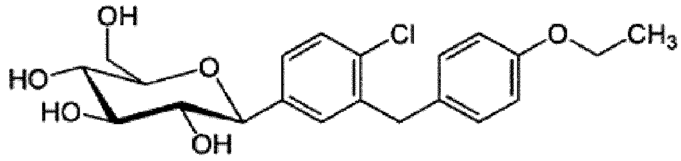

formula 1,

and at least one polymer selected from the group consisting of polyvinyl pyrrolidone (PVP), polyvinyl alcohol (PVA), polyacrylic acid (PAA), poly(ethylene glycol) (PEG), poly(ethylene oxide) (PEO), hydroxypropyl cellulose (HPC), hydroxypropyl methyl cellulose (HPMC), copovidone, hypromellose acetate succinate (AQOAT), polyacrylates and mixtures thereof.

(4) The solid dispersion according to any one of items 1 to 3, wherein the weight ratio of dapagliflozin and the at least one polymer is from 1:10 to 10:1, preferably from 1:1 to 1:10.
In a further preferred embodiment, the weight ratio of dapagliflozin and the at least one polymer is about 1:1, 1:2, 1:3, 1:4, or 1:5. In a further preferred embodiment, the weight ratio of dapagliflozin and the at least one polymer is about 1:2.

(5) The solid dispersion according to at least one of items 1 to 4, wherein the dapagliflozin is stable in the amorphous state upon storage, optionally upon storage under stress conditions, for example at 40 °C and 75 % humidity.

(6) The solid dispersion according to at least one of items 1 to 5, wherein said dapagliflozin and said polymer do not form an inclusion complex, in particular said polymer is not a cyclodextrin or cyclodextrin derivative such as a substituted cyclodextrin, in particular said dapagliflozin and said polymer do not form a dapagliflozin-cyclodextrin inclusion complex or dapagliflozin-cyclodextrin derivative inclusion complex.

(7) The solid dispersion according to at least one of items 1 to 6, wherein said polymer comprises polymer chains having more than 10, more than 100, or more than 1.000 monomer units.

(8) The solid dispersion according to at least one of items 1 to 7, wherein said polymer is not a cyclic polymer and/or is a linear or branched polymer. Preferably, said polymer is not a cyclic polymer. Also preferably, said polymer is a linear polymer. Also preferably, said polymer is a branched polymer.

(9) The solid dispersion according to at least one of items 1 to 6, wherein dapagliflozin is present in its free form, in particular, dapagliflozin is not present in the form of solvates, hydrates or salts. The term "free form" within the meaning of the present invention denotes that dapagliflozin is present in its pure form, e.g. that no solvate or hydrate of dapagliflozin is present. Preferably, dapagliflozin is not in the form of its propylene glycol hydrate (in particular, (S)-PG (form SC-3) la as defined in Table 1 of WO 2008/002824).

(10) The solid dispersion according to at least one of items 1 to 9, wherein the solid dispersion does not contain crystalline portions of dapagliflozin, in particular no crystalline dapagliflozin portions can be detected by X-ray powder diffraction measurement. Preferably, the solid dispersion does not contain crystal structures of dapagliflozin, including the crystal structures defined in WO 2008/002824.

(11) The solid dispersion according to any of items 1 to 10, being in the form of granules. In other words, the granules comprise said solid dispersion.
According to the invention, the term "granule" means a small compact particle of a substance (e.g., including powder form).

(12) The solid dispersion according to item 11, wherein the granules comprise a carrier. In other words, the granules comprise or consist of carrier particles with solid dispersion, wherein the solid dispersion can be on the surface of the carrier particles and between them.

(13) The solid dispersion according to item 12, wherein the carrier is selected from the group consisting of water insoluble polymers; inorganic salts; sugars such as lactose; cellulose and cellulose derivatives; starch; sugar alcohols; inorganic oxides; preferably cellulose, such as microcrystalline cellulose, e.g. Avicel®; and sugars, such as lactose (monohydrate or anhydrous).
Such carriers are known to a skilled person, and some are described in more detail herein.

(14) The solid dispersion according to any of items 1 to 10, being in the form of a powder.
The preferred particle size distribution is described herein below.

(15) The solid dispersion according to item 14, wherein the powder does not comprise a carrier particles, preferably the powder does not comprise a carrier particles as defined in item 13.

(16) Pharmaceutical composition comprising the solid dispersion according to any one of items 1 to 15 and one or more pharmaceutical excipients, wherein said excipients are selected from the group consisting of fillers, disintegrants, binders, lubricants, glidants,surfactants, wetting agents, film-forming agents and coating materials, sweeteners, flavoring agents, and coloring agents such as example pigments.

(17) Process for the preparation of the solid dispersion as defined in any of items 1 to 15, comprising:

a) providing a solution of dapagliflozin and at least one suitable polymer in a suitable solvent or mixture of solvents, wherein the solvent or mixture of solvents is preferably selected from the group consisting of water, halogenated hydrocarbon, C1-C4 alcohol, C3-C6 ketone, organic ether, organic ester or mixtures thereof, and are more preferably selected from the group consisting of ethanol, water, acetone, isopropanol or mixtures thereof;
b) optionally spraying or dispersing the solution of step (a) onto carrier particles as defined in item 13 to form granules, preferably during low shear, high shear or fluid bed granulation process, and
c) evaporating the solvent, wherein the evaporation step is preferably carried out by fluid bed drying, spray drying, freeze drying (lyophilisation), vacuum drying, tray drying, microwave drying or other processes that are known to a skilled person to result in solvent evaporation, thereby resulting in the formation of solid dispersion.

The expression "suitable solvent" as used herein refers to a solvent or mixture of solvents which is able to (substantially completely) dissolve dapagliflozin and the at least one suitable polymer, but does not, or at least not completely, dissolve the carrier particles.
Preferably, the suitable polymer is a linear water soluble polymer, and/or the solvent or mixture of solvents is selected from the group consisting of water, halogenated hydrocarbon, C1-C4 alcohol, C3-C6 ketone, organic ether, organic ester or mixtures thereof, and is more preferably selected from the group consisting of ethanol, water, acetone, isopropanol, or mixtures thereof.

(18) Process for the preparation of a pharmaceutical composition, wherein dapagliflozin is present in the pharmaceutical composition only as amorphous dapagliflozin, comprising:

a) providing a solution of dapagliflozin of formula 1

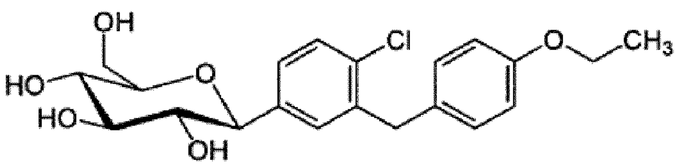

formula 1

and optionally at least one suitable polymer in a suitable solvent or mixture of solvents, wherein the at least one polymer is preferably defined as in item 17;
b) optionally spraying or dispersing the solution of step (a) onto carrier particles as defined in item 13 to form granules, preferably during low shear, high shear or fluid bed granulation process,
c) evaporating the solvent, wherein the evaporation step is preferably carried out by fluid bed drying, spraying, freeze drying (lyophilisation), vacuum drying, tray drying, microwave drying or other suitable processes that are known to a skilled person to result in solvent evaporation; and
d) blending the obtained composition of steps b) or c) with one or more pharmaceutically acceptable excipients.

The solvent or mixture of solvents can be selected from the group consisting of water, halogenated hydrocarbon, C1-C4 alcohol, C3-C6 ketone, organic ether, organic ester or mixtures thereof, and are more preferably selected from the group consisting of water, ethanol, acetone, isopropanol or mixtures thereof.

(19) Process for the preparation of a pharmaceutical composition, comprising the steps of

a) providing a solid dispersion of the present invention by applying the steps defined in one of items 17-18, and
b) blending said solid dispersion of step (a) with one or more pharmaceutically acceptable excipients,

The pharmaceutical composition can then be used for preparing a dosage form, for example by compressing the mixture of step (b) into tablet cores and optionally coating said tablet cores.

(20) Solid solution, pharmaceutical composition or granules obtainable by or obtained by the process according to items 17 to 19.

(21) Solid dispersion according to any of items 1 to 15 or a pharmaceutical composition according to item 16, wherein the API dapagliflozin is the only API being present.

(22) Adsorbate comprising dapagliflozin ((2S,3R,4R,5S,6R)-2-[4-chloro-3-(4-ethoxybenzyl)phenyl]-6-(hydroxymethyl)-tetrahydro-2H-pyran-3,4,5-triol) of formula 1 adsorbed onto the surface of a substrate

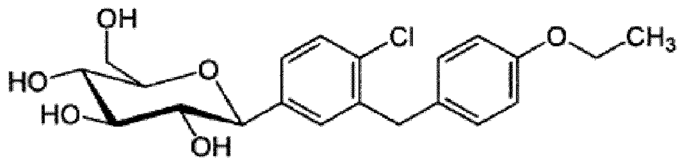

formula 1,

wherein dapagliflozin is substantially amorphous and wherein the substrate is selected from the group consisting of

(a) an inorganic oxide;
(b) water insoluble inorganic salt;
(c) water insoluble polymer; and
(d) an activated carbon.

(23) The adsorbate according to item 22, wherein

(i) the inorganic oxide is selected from the group consisting of $SiO_2$, $TiO_2$, $ZnO_2$, ZnO, $Al_2O_3$ and zeolite, preferably the inorganic oxide is $SiO_2$, and $CaCO_3$, $Ca_2(PO4)_2$
(ii) the water insoluble polymer is selected from the group consisting of cross-linked polyvinyl pyrrolidinone, cross-linked cellulose acetate phthalate, cross-linked hydroxypropyl methyl cellulose acetate succinate, microcrystalline cellulose, polyethylene/polyvinyl alcohol copolymer, polyethylene/polyvinyl pyrrolidinone copolymer, cross-linked carboxymethyl cellulose, sodium starch glycolat, and cross-linked styrene divinyl benzene, preferably the water insoluble polymer is microcrystalline cellulose, and/or
(iii) wherein the activated carbon is selected from the group consisting of polyimides, polyarylonitrile, phenolic resins, cellulose acetate, regenerated cellulose, and rayon.

(24) The adsorbate according to item 22 or 23, wherein the substrate is selected from the group consisting of silicon dioxide, e.g. colloidal or fumed silicon dioxide or porous silica, and cellulose, preferably microcrystalline cellulose.

(25) The adsorbate according to items 22 to 24, wherein dapagliflozin is not in the form of particles.
The presence (or absence) of dapagliflozin particles can be assessed by any suitable method that is known to a person skilled in the art, for instance by Raman imaging.

(26) The adsorbate according to any of items 22 to 25, wherein said dapagliflozin is associated with the substrate. The substrate according to the present invention may be a particulate and/or porous substrate, which means that

this substrate has an outer and/or inner surface onto which the API can be adsorbed. This means that if the substrate has pores, these pores are filled by the dapagliflozin. Furthermore, the substrate according to the present invention does not, at least not essentially, change its morphology during and after the adsorption of the API, i.e. the physical shape and outer structure of the adsorbate corresponds to, at least essentially corresponds to, the physical shape and outer structure of the substrate alone.
Preferred adsorbates and preferred particulate and/or porous substrates are described herein. The porosity can be determined according to DIN EN 623-2, wherein the porosity is preferably at least 20 %, 30 %, 40 %, 50 % or 60 %. Also preferred, the porosity is in the range of between 10-70 %, further preferred between 20-70 %, even further preferred between 30-70 % or between 40-70 %.

(27) The adsorbate according to any of items 22 to 26, wherein said dapagliflozin is present in its free form, in particular, dapagliflozin is not present in the form of solvates, hydrates or salts.

(28) The adsorbate according to items 22 to 27, wherein the adsorbate does not contain substantial amounts, preferably does not contain noticeable amounts of, crystalline portions of dapagliflozin as e.g. measurable upon X-ray powder diffraction measurement.

(29) The adsorbate according to items 22 to 28, wherein the amount of dapagliflozin in the adsorbate is in the range of 0.01 to 40 wt.-%, preferably in the range of 0.1 to 30 wt.-%, more preferably in the range of 1 to 30 wt.-%, and even more preferably in the range of 10 to 30 wt.-% (respectively in % by weight relative to the whole adsorbate).

(30) Process for the preparation of an adsorbate according to any one of items 22 to 29, comprising:

a) combining a solution of dapagliflozin of formula 1

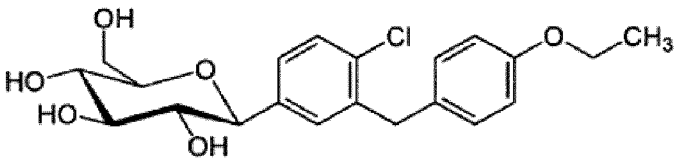

formula 1

in a solvent or mixture of solvents with a substrate, wherein the solvent or mixture of solvents is optionally selected from the group consisting of halogenated hydrocarbon, C5-C9 hydrocarbon, C1-C4 alcohol, C3-C6 ketone, organic ether, organic ester and mixtures thereof;
b) removing (or evaporating) the solvent or mixture of solvents under reduced pressure to form the adsorbate.

A too fast evaporation of the solvent leads to the formation of e.g. coprecipitates of the API, which would not be adsorbed on the substrate.

(31) Process for the preparation of a pharmaceutical composition comprising an adsorbate according to any one of items 22 to 29, comprising:

a) providing a mixture of the adsorbate prepared by the process of item 30, and at least one pharmaceutically acceptable excipient;
b) optionally fine-milling and/or sieving the mixture obtained in step a);
c) formulation of the mixture of step a) or b) into a pharmaceutical composition by dry formulation.

(32) Pharmaceutical composition comprising the adsorbate according to any one of items 22 to 29 or obtainable by the process according to item 30, and one or more pharmaceutically excipients e.g. selected from the group consisting of fillers, disintegrants, binders, lubricants, and surfactants.

(33) Pharmaceutical composition according to item 32, wherein the amount of the adsorbate in the pharmaceutical composition is in the range of 1 to 95 wt.-%, preferably in the range of 5 to 90 wt.-%, more preferably in the range of 10 to 70 wt.-% and even more preferably in the range of 20 to 50 wt.-% (respectively in % by weight relative to the whole pharmaceutical composition).

(34) The pharmaceutical composition according to any one of the preceding items, wherein the pharmaceutical composition is a dosage form, preferably a compressed dosage form, more preferably a tablet, even more preferably an immediate release tablet, and/or wherein dapagliflozin is present in the pharmaceutical composition only as amorphous dapagliflozin.

(35) The pharmaceutical composition according to any one of the preceding items, wherein the pharmaceutical composition is to be administered to patients in a country having an area with an Af or an Am climate, preferably an Af climate, according to the Köppen-Geiger climate classification.

(36) The pharmaceutical composition according to any one of the preceding items, wherein said pharmaceutical composition is packaged in a packaging material having a moisture vapour transmission rate of at least 0.4 g $m^{-2}$ $d^{-1}$ as measured according to standard DIN 53122-1, said packaging material preferably being made from polypropylene, polyvinylidenchloride and/or polyvinylchloride or being an Al-Al push through packaging-blister.

(37) The pharmaceutical composition according to any of the preceding claims, wherein the content uniformity, indicated in terms of acceptance value (AV), preferably as defined by the legend of Table 2, of said pharmaceutical composition being packaged in a packaging material such as a blister containing N tablets is below 15, preferably below 10.

(38) The pharmaceutical composition according to any of the preceding claims, wherein, when carrying out dissolution testing, at a time point of 5 minutes more than 80%, preferably more than 85% of the dapagliflozin is dissolved, wherein the dissolution testing is carried out by applying the following parameters: 500 ml of dissolution medium 0.1 M HCl, Apparatus 2 ant 50 rpm, peak vessel, 37°C.

(39) Solid solution, adsorbate or pharmaceutical composition according to any one of the preceding items for use in the treatment of diseases related to hypoglycemia, preferably type II diabetes mellitus, optionally in patients in a country having an area with an Af or an Am climate according to the Köppen-Geiger climate classification.

Definitions

Where the term "comprising" is used in the present description and claims, it does not exclude other elements or steps. For the purposes of the present invention, the term "consisting of" is considered to be a preferred embodiment of the term "comprising". If hereinafter a group is defined to comprise at least a certain number of embodiments, this is also to be understood to disclose a group which optionally consists only of these embodiments.

Where an indefinite or definite article is used when referring to a singular noun e.g. "a" or "an", "the", this includes a plural of that noun unless something else is specifically stated.

The terms "about" or "substantially" in the context of the present invention denote an interval of accuracy that the person skilled in the art will understand to still ensure the technical effect of the feature in question. The term typically indicates deviation from the indicated numerical value of $\pm$10%, and preferably $\pm$5%.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The term "oral solid dosage form" as used herein denotes solid preparations (e.g. tablets) for oral administration each containing a single dose of one or more active substances.

In the context of the present invention, the term "dissolution rate" relates to the percentage (weight-%) of dapagliflozin in a pharmaceutical composition which is dissolved after a defined number of minutes if the dapagliflozin-containing composition is subjected to dissolution conditions using an USP 2 apparatus with a stirrer speed of 50 rpm and a test temperature of 37 °C with a dissolution medium of 500 ml of a 0.1 M HCl solution, in peak vessel. Alternatively, where indicated, a dissolution medium of 500 ml of a phosphate buffer at pH 6.8 was used.

In the context of the present invention, the term "amorphous dapagliflozin" indicates that the dapagliflozin is present in the composition (e.g. solid dispersion, adsorbate or pharmaceutical composition) of the present invention in substantially amorphous state. "Substantially" amorphous denotes that 90 %, preferably 95 % or 97 %, more preferably all of the dapagliflozin being present in the solid solution, on the adsorbate or in the pharmaceutical composition is amorphous. In other words, an "amorphous" dapagliflozin composition denotes a dapagliflozin-containing composition, which does not contain substantial amounts, preferably does not contain noticeable amounts, of crystalline portions of dapagliflozin as e.g. measurable upon X-ray powder diffraction analysis. In order to assess whether the dapagliflozin-containing composition according to the present invention comprises only amorphous dapagliflozin, the X-ray powder

diffraction pattern of the dapagliflozin-containing composition is compared to the X-ray powder diffraction pattern of a placebo-composition, i.e. the composition without dapagliflozin. If the respective patterns of dapagliflozin-containing composition and placebo-composition correspond to each other, dapagliflozin is present in amorphous form only.

Within the meaning of the present invention, the term "solid dispersion" (or "solid solution"), denotes a state where most of the dapagliflozin, preferably 90%, 95% or all of the dapagliflozin of the solid dispersion, is homogeneously molecularly dispersed in a solid polymer matrix. The API dapagliflozin is not present in form of inclusion complexes of a polymer and API. Thus, the polymer suitable for and used for the matrix is a polymer that does not provide molecular cavities into which the dapagliflozin is entrapped. This is contrary to API -polymer - complexes where the API is entrapped or intercalated into molecular cavities of polymers that provide such cavities.

Hence, in the context of the present invention, the term "solid dispersion", preferably solid solution, relates to a molecular dispersion where the API (active pharmaceutical ingredient) and polymer molecules are uniformly but irregularly dispersed in a non-ordered way. In other words, in a solid dispersion, the two components (polymer and API) form a homogeneous one-phase system, where the particle size of the API in the solid dispersion is reduced to its molecular size. In a preferred embodiment, in the solid dispersion according to the present invention no chemical bonds can be detected between the API and the polymer. In order to arrive at such a solid dispersion, preferably solid solution, it is required to have a substantial amount of API (in the context of the present invention the API is dapagliflozin) dissolved in a suitable solvent at least at one time point during preparation of said solid dispersion. Only if this prerequisite is fulfilled, a solid dispersion within the meaning of the present invention can be generated. A "substantial amount" of API means that at least 80%, preferably at least 90%, and more preferably at least 95% of API are dissolved in a suitable solvent. In a further preferred embodiment, the entire API is dissolved when preparing the solid dispersion.

In order to characterize the physical nature of solid dispersions, techniques such as thermal analysis (such as cooling curve, thaw melt, thermo microscopy and DTA methods), x-ray diffraction, microscopic methods, spectroscopic methods, dissolution rate, and thermodynamic methods can be used. It is also possible to use two (or even more) of the above listed methods in order to obtain a complete picture of the solid dispersion system, if need be.

In a preferred embodiment of the present invention, the solid dispersion is a solid solution.

Contrary to the solid dispersion of the present invention, the term "inclusion complex" denotes an ordered arrangement of API molecules inside cyclic polymer cavities. In inclusion complexes, essentially a 1:1 ratio of cyclic polymers : API molecules exists, with detectable chemical bonds between the API molecule and the cyclic polymer. This is in contrast to the solid dispersion according to the present invention, where the amount of polymer is much higher than the amount of API.

The expression "adsorbate", as used herein, specifies that the API is, preferably evenly, and preferably homogeneously, distributed on the inner and/or outer surface of the particulate substrate. The distribution of the API on the surface of a carrier or substrate (e.g. within microcrystalline cellulose) can be analyzed for instance by Raman imaging. The API of the present invention is preferably homogeneously distributed within the composite with the carrier (such as microcrystalline cellulose) in a layer, with this layer preferably having a thickness of about 1 μm to 50 μm, or, for instance in the case of e.g. microcrystalline cellulose, of about 5 μm to 15 μm.

Within the meaning of the present invention, the expression "adsorbed onto the surface of a substrate" denotes that API (here: dapagliflozin) is deposited on the inner and/or outer surface of a suitable substrate, wherein the API is in its free form, and no API particles are formed on the substrate. Further, the API is not present in the form of solvates, hydrates or salts.

Within the meaning of the present invention, the term "substrate" refers to a solid support onto which a dapagliflozin solution can be applied. The application can for instance be carried out by preferably slowly removing and/or evaporating the solvent or mixture of solvents.

In the context of the present invention, the term "chemical stability" means that the sum of all degradation products derived from dapagliflozin is below 2 percent, preferably below 0.5% of the total amount of dapagliflozin after storage at defined conditions. Analysis and detection of degradation products is performed by HPLC. The term "chemical stability" may also mean "polymorphic stability". In the context of the present invention, the term "polymorphic stability" means that dapagliflozin does not convert to a crystalline form, as determined by XRPD.

Detection of other polymorphic forms of dapagliflozin or of amorphous dapagliflozin can be done by XRPD measurements.

In the context of the present invention, the presence of amorphous dapagliflozin is determined by XRPD. The absence of crystalline particles of (or in) a given sample can be determined by subjecting the sample XRD and comparing the sample to placebo.

In the context of the present invention, the term "storage under stress condition" means that samples were exposed in open atmosphere to elevated temperature 40°C and high humidity conditions of 65% relative humidity for 21 days (up to 1 month or 3 months). Increase in degradation product(s) on storage under stress condition compared to initial state was evaluated in order to determine stability properties.

In the context of the present invention, the term "storage under accelerated condition" means that samples

were exposed in non-permeable container to elevated temperature 40°C and high humidity conditions of 75% relative humidity in accordance with ICH guidance, for period up to 3 months. Increase in degradation product(s) on storage under accelerated conditon compared to initial state was evaluated in order to determine stability properties.

The Köppen-Geiger classification is one of the most widely used climate classification systems. It combines average annual and monthly temperatures and precipitation, and the seasonality of precipitation in an area. Examples of countries having an area with an Af climate are Brazil, Indonesia, Mexico, Puerto Rico, Zaire, to name but a few. Examples of countries having an area with an Am climate are Brazil, Indonesia, Mexico, Cuba, the USA, Zaire, India, China, Birma, to name but a few.

Particle size distribution may be described using quantiles, e.g. D5%, D 10%, D50%, D90%, D95% and D98%. As used herein, "particle size distribution" means the cumulative volume size distribution of equivalent spherical diameters as determined by laser diffraction method

(e.g., in a Malvern Mastersizer). Methods of determining the particle size distribution are known to the skilled person, e.g. measurements including dispersing of measured material in non-solvent (by using mixing or ultrasound).

In the context of the present invention "slightly hygroscopic" means that a tested substance displays a mass increase of at most 2%, but at least 0.2% when tested by the hygroscopicity assay and using the environmental conditions according to 5.11. of the European Pharmacopoeia 7.0.

In the context of the present invention "non-hygroscopic" means that a tested substance displays a mass increase of at most 0.2% when tested by the hygroscopicity assay and using the environmental conditions according to 5.11. of the European Pharmacopoeia 7.0.

In the context of the present invention, a "carrier" within the meaning of the present invention is also referred to herein as "particles of a carrier" or "carrier particles".

## Detailed description of the invention

The present invention is now described in more detail by preferred embodiments and examples, which are however presented for illustrative purpose only and shall not be understood as limiting the scope of the present invention in any way.

Pure amorphous dapagliflozin has a low glass transition temperature (around 40°C). Further, it is hygroscopic, which means that it absorbs around 6% of water at 80% relative air humidity (RH). The sorption and desorption during the first cycle exhibit hysteresis. Desorption is incomplete, and around 3% of water is irreversibly absorbed during the first cycle, which results in the change of consistence from powder to semisolid. These properties, i.e. the low glass transition temperature and hygroscopicity, have a negative impact on processibility of dapagliflozin, going along with a negative influence on important properties of pharmaceutical compositions comprising dapagliflozin which contribute to the desired therapeutic effect, such as content uniformity, storage stability or dissolution profile. Other forms of dapagliflozin, e.g. dapagliflozin propylene glycol hydrate is only slightly hygroscopic, as it absorbs only around 0.8% of water at 80% RH.

As can be seen from Example 1, providing dosage forms containing dapagliflozin in its free from and having a very good dissolution profile is more demanding than for instance when using dapagliflozin solvate, such as the form of propylene glycol solvate e.g. disclosed in WO 2008/002824 or WO 2008/116179.

It was surprisingly found that dapagliflozin being present in the amorphous form in pharmaceutical formulations according to the present invention, in particular pharmaceutical compositions comprising a solid dispersion or adsorbates according to the present invention, can provide an overall improved performance e.g. with regard to processability, content uniformity, (storage) stability, and/or dissolution profile when compared with products obtained by prior art manufacturing techniques.

Additionally, it was surprisingly found that when dapagliflozin was dissolved in a solvent together with a suitable polymer and the obtained solution was sprayed onto circulating powders in a fluid bed apparatus, in particular when using non-active excipient carrier (i.e. free of active substance), the dissolution was surprisingly improved in comparison with common prior art preparation techniques. It was also surprisingly found that when dapagliflozin was prepared as adsorbate on an inert substrate and later blended with excipients the dissolution was surprisingly improved and increased powder handling properties were achieved.

Therefore, in one aspect, the present invention refers to an amorphous solid dispersion, preferably solid solution, of at least one suitable polymer and dapagliflozin ((2S,3R,4R,5S,6R)-2-[4-chloro-3-(4-ethoxybenzyl)phenyl]-6-(hydroxymethyl)-tetrahydro-2H-pyran-3,4,5-triol) of formula 1

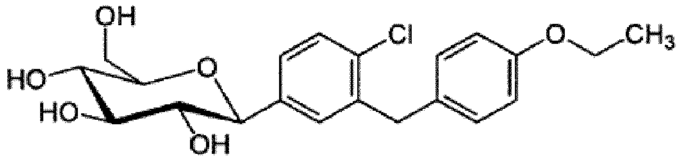

formula 1.

The API dapagliflozin is not present in form of inclusion complexes of matrix polymer and API. Thus, the polymer used for the matrix is a polymer that does not provide molecular cavities into which the dapagliflozin is entrapped. This is contrary to API -polymer complexes where the API is entrapped or intercalated into molecular cavities of polymers that provide such cavities.

Any suitable methods that are known to a person skilled in the art can be used for identifying suitable polymers.

In a preferred embodiment, the solid dispersion of the present invention is substantially homogeneous.

The at least one suitable polymer being present in the solid dispersion can be selected from the group consisting of polyvinyl pyrrolidone (PVP), polyvinyl alcohol (PVA), polyacrylic acid (PAA), poly(ethylene glycol) (PEG), poly(ethylene oxide) (PEO), hydroxypropyl cellulose (HPC), hydroxypropyl methyl cellulose (HPMC), copovidone, hypromellose acetate succinate (AQOAT), polyacrylates and mixtures thereof.

In a preferred embodiment, the at least one polymer is preferably selected from the group consisting of PVP, PVA, HPC and HPMC.

In an even more preferred embodiment, the at least one polymer is selected grom the group consisting of polyvinyl pyrrolidone (PVP) and polyvinyl alcohol (PVA).

In a further embodiment of the solid dispersion of the present invention, the weight ratio of dapagliflozin and the at least one polymer can be from 1:10 to 10:1, preferably from 1:1 to 1:10. In a preferred embodiment, the weight ratio of dapagliflozin and the at least one polymer is about 1:2. The weight ratio of the two components depends on the final size of the dosage form.

Preferably, the dapagliflozin comprised in the solid dispersion described above is stable in the amorphous state upon storage, optionally upon storage under stress and accelerated conditions, for example at 40 °C and 75 % humidity for 1 month, 2 months or 3 months, wherein the sum of impurities determined by HPLC remains below 0.10 or even 0.05.

It is further preferred that in the solid dispersion according to the present invention, dapagliflozin and the above disclosed suitable polymer do not form an inclusion complex. In a preferred embodiment, said polymer is not a cyclodextrin or cyclodextrin derivative such as a substituted cyclodextrin, preferably said dapagliflozin and said polymer do not form a dapagliflozin-cyclodextrin inclusion complex or dapagliflozin-cyclodextrin derivative inclusion complex.

By avoiding the formation of e.g. inclusion complexes or other solutions comprising dapagliflozin which are not the solid dispersion according to the present invention, but rather by applying solid dispersions according to the present invention, an improved process e.g. with regard to time and costs can be reached. This is because excipients and/or process techniques that would otherwise be required are not necessary. Further, pharmaceutical compositions, e.g. dosage forms such as tablets or capsules, can be manufactured that exhibit improved dissolution properties, content uniformity, and appropriate storage properties such as stability when compared to pharmaceutical dosage forms which have been prepared conventionally.

The polymer used for preparing solid dispersions of the present invention can comprise polymer chains having more than 10, more than 100, or more than 1.000 monomer units. Further, the polymer preferably has not more than 50.000 monomer units, and/or has an average molecular weight of at least 1.000 or at least 10.000 Daltons, but not more than 3.000.000 Daltons. Preferably, said polymer is not a cyclic polymer and/or is a linear or branched polymer. In a preferred embodiment, the polymer used for preparing solid dispersions of the present invention comprises polymer chains having between 10, 100, or 1.000 monomer units and 50.000 monomer units. In a further preferred embodiment, the polymer used for preparing solid dispersions of the present invention comprises polymer chains having between 100 monomer units and 10.000, 25.000 or 50.000 monomer units. In a further preferred embodiment, the polymer used for preparing solid dispersions of the present invention has an average molecular weight of between 1.000, 10.000 or 100.000 Daltons and 3.000.000 Daltons. In a further preferred embodiment, the polymer used for preparing solid dispersions of the present invention has an average molecular weight of between 10.000 Daltons and 250.000, 500.000, 1.000.000, 2.000.000 or 3.000.000 Daltons.

According to the present invention, dapagliflozin is present in the solid dispersion preferably in its free form. In particular, dapagliflozin is not present in the form of solvates, hydrates or salts. Dapagliflozin is also preferably used in its free form for preparing the solid dispersions of the invention. In contrast to prior art formulations that contain dapagliflozin not in its free (pure) form, but for instance contain dapagliflozin and propylene glycol hydrate, herein disclosed are pure dapagliflozin formulations. The use of dapagliflozin in its free form can improve the production process e.g. in respect of production time, production yield, and costs by e.g. reducing the number of production steps. For instance, the process according to the present invention does not require the preparation of dapagliflozin propylene glycol solvate

hydrate.

It is further preferred that the solid dispersion does not contain crystalline portions that could be associated with crystalline dapagliflozin, in particular no crystalline portions can be detected by X-ray powder diffraction measurement, i.e. no peaks that could be allocated to crystalline dapagliflozin, are observed. Crystalline forms of dapagliflozin and their characteristic XRPD peaks are known to the skilled person, e.g. from WO 2008/002824.

In a further embodiment it is also possible that the solid dispersion is applied to a carrier, also referred to herein as "particles of a carrier" or "carrier particles". Such a carrier can for instance be selected from the group consisting of water insoluble polymers; inorganic salts; sugars; cellulose and cellulose derivatives; starch; sugar alcohols; inorganic oxides; preferably cellulose, such as microcrystalline cellulose, e.g. Avicel®; and sugars, such as such as lactose (monohydrate or anhydrous). Preferably, the carrier is water insoluble. If the solid dispersion is applied to carrier particles, granules can be formed. The preferred particle size distribution of said particles is described elsewhere herein. Preferably, the solid dispersion is applied onto the carrier particles by spraying or dispersing the solution of dapagliflozin and at least one suitable polymer in a suitable solvent or mixture of solvents as described herein onto said carrier particles. By spraying or dispersing said solution, a granulation process is carried out. This granulation process preferably is a low shear, high shear or fluid bed granulation process.

Thus, the invention also refers to a process for the preparation of a solid dispersion according to the present invention, comprising

a) providing a solution of dapagliflozin and at least one suitable polymer in a suitable solvent or mixture of solvents, wherein the solvent or mixture of solvents is preferably selected from the group consisting of water, halogenated hydrocarbon, C1-C4 alcohol, C3-C6 ketone, organic ether, organic ester or mixtures thereof, and are more preferably selected from the group consisting of ethanol, water, acetone, isopropanol or mixtures thereof;
b) optionally spraying or dispersing the solution of step (a) onto carrier particles as defined in item 13 to form granules, preferably during low shear, high shear or fluid bed granulation process,
c) evaporating or removing the solvent, wherein the evaporation step is preferably carried out by fluid bed drying, spray drying, freeze drying (lyophilisation), vacuum drying, tray drying, microwave drying or other processes that are known to a skilled person to result in solvent evaporation, thereby resulting in the formation of solid dispersion.

As described above, if step b) is carried out, granules are obtained. If step b) is not carried out, which means that the solid dispersion is not applied onto carrier particles, then the solvent is removed (or evaporated), thus resulting in a powder.

The present invention also refers to a process for the preparation of a pharmaceutical composition, wherein dapagliflozin is present in the pharmaceutical composition only as amorphous dapagliflozin, comprising:

a) providing a solution of dapagliflozin of formula 1

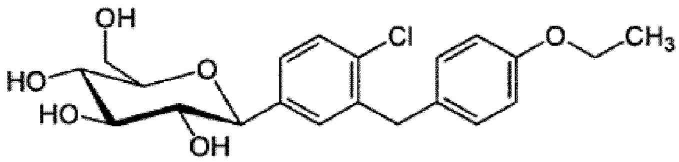

formula 1

and optionally at least one suitable polymer in a suitable solvent or mixture of solvents, wherein the at least one polymer is selected as described herein;
b) optionally spraying or dispersing the solution of step (a) onto carrier particles as defined in item 13 to form granules, preferably during low shear, high shear or fluid bed granulation process,
c) evaporating or removing the solvent, wherein the evaporation or removing step is preferably carried out by fluid bed drying, spraying process, freeze drying (lyophilisation), vacuum drying, tray drying, microwave drying or other suitable processes that are known to a skilled person to result in solvent evaporation; and
d) blending the obtained composition of steps b) or c) with one or more pharmaceutically acceptable excipients.

In a preferred embodiment, the solvent evaporating or removing step is carried out in a fluid bed dryer using suitable conditions that are known to a person skilled in the art.

The evaporation of the solvent may be performed simultaneously with spraying/dispersion the solution over excipients (fluid bed dryer) or subsequently (first dispersing the solution onto excipients in high shear and afterwards drying - removing of the solvent in fluid bed, vacuum, tray dryer, microwave etc.

The fluid bed drying, spraying-, freeze-drying-, vacuum drying- tray drying, microwave-drying-, and blending-process that are referred to herein are processes that are well known to a person skilled in the art and thus can be carried out by a skilled person following suitable guidelines.

Spray-drying processes can for example be performed in fluid bed granulators such as top spray granulators or spray drying machines. High-shear granulation can for example be performed in high shear granulators such as Gral 10, and fluid bed granulation can for instance be carried out in a Glatt GPCG fluid bed granulator, using suitable conditions. For instance, fluid bed granulation can be carried out using the following conditions: inlet air temperature: 30-80°C; spray rate: 5-50g/min. High shear mixing can be carried out by using the following conditions: mixing speed: 200-500RPM; Mixing time: 1-10 min..

In order to provide the solution of dapagliflozin and the at least one polymer in a suitable solvent (step a)), suitable methods that are known to a person skilled in the art can be used. For instance, the polymer can be dissolved in a suitable solvent or mixture of solvents, and dapagliflozin is also dissolved in this solvent or mixture of solvents. The order of these steps can also be reversed.

The solvent or mixture of solvents can be selected from the group consisting of water, halogenated hydrocarbon, C1-C4 alcohol, C3-C6 ketone, organic ether, organic ester or mixtures thereof, and are more preferably selected from the group consisting of ethanol, water, acetone, isopropanol or mixtures thereof.

The suitable polymer can be a linear water soluble polymer, and/or the solvent or mixture of solvents is preferably selected from the group consisting of water, halogenated hydrocarbon, C1-C4 alcohol, C3-C6 ketone, organic ether, organic ester or mixtures thereof, and are more preferably selected from the group consisting of ethanol, water, acetone, isopropanol, or mixtures thereof.

The spray-drying-, drying- and blending-process are processes that are well known to a person skilled in the art and can be carried out by a skilled person following suitable known guidelines such as the "Encyclopedia of pharmaceutical technology".

In a further embodiment, a suitable hot melt procedure (hot melt extrusion) that is known to a skilled person can be applied in order to provide a solid dispersion: The dapagliflozin and the at least one polymer are heated and mixed together. Hot melt procedures are known to a skilled person and are for instance carried out in a hot melt extruder according to the following protocol: Polymer and API are premixed and fed into the extruder. In this extruder, the material is heated until softening and then extruded through a matrix. Afterwards, the obtained extrudate is cooled down and chopped or milled. It has to be noted that this hot melt extrusion process in step a) can be carried out not only when preparing the granules, but also e.g. when preparing the solid dispersion or the pharmaceutical composition as described elsewhere herein.

The present invention also refers to a process for the preparation of a pharmaceutical composition, comprising the steps of

a) providing a solid dispersion of the present invention by applying the steps defined above, and
b) blending said solid dispersion of step (a) with one or more pharmaceutically acceptable excipients.

The pharmaceutical composition can then be used for preparing a dosage form, for example by compressing the mixture of step (b) into tablet cores and optionally coating said tablet cores.

The present invention also refers to a solid dispersion, granules or pharmaceutical composition obtainable by the process described herein. Preferably, there is no further API present that is known to be used for treating type II diabetes. In a further preferred embodiment, in the solid dispersion, granule, or pharmaceutical composition described herein, dapagliflozin is the only API being present. Thus, step b) of the above-described processes is preferably carried out in the absence of further pharmaceutically active ingredients that are known to be used in the treatment of type II diabetes, such as metformin, more preferred, step b) is carried out in the absence of any further API. In other words, in a preferred embodiment, dapagliflozin is the only API that is present in the solid dispersion, granules, adsorbates, and/or pharmaceutical compositions according to the present invention.

The granules, pharmaceutical compositions and solid dispersions that are obtained or obtainable according to a process of the present invention exhibit improved properties such as an improved content uniformity when compared to the respective properties (e.g. content uniformity) of granules, pharmaceutical compositions and solid dispersions that have been prepared according to conventional methods. Content uniformity can be determined according to any suitable method that is known to a skilled person, e.g. according to the European Pharmacopoeia (Ph. Eur.) 2.9.40.

The present invention also refers to an adsorbate comprising dapagliflozin ((2S,3R,4R,5S,6R)-2-[4-chloro-3-(4-ethoxybenzyl)phenyl]-6-(hydroxymethyl)-tetrahydro-2H-pyran-3,4,5-triol) of formula 1 adsorbed onto the surface of a substrate

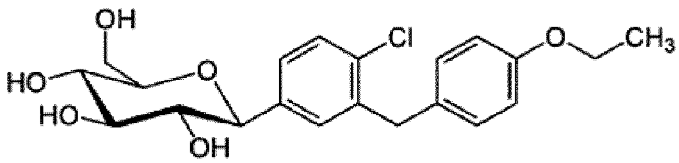

formula 1,

wherein dapagliflozin is substantially amorphous and wherein the substrate is selected from the group consisting of

(a) an inorganic oxide;
(b) water insoluble inorganic salt;
(c) water insoluble polymer; and
(d) an activated carbon.

wherein (a), (b) and (c) are preferred.
It has been surprisingly found that the adsorbate according to the present invention provides for that the API dapagliflozin which has a good water solubility (about 1mg/ml) exhibits a good processibility when being in the form of adsorbates. Preferably, in the adsorbate, the dapagliflozin is not present in the form of particles.
The inorganic oxide can be selected from the group consisting of $SiO_2$, $TiO_2$, $ZnO_2$, ZnO, $Al_2O_3$ and zeolite, and/or wherein the water insoluble polymer is selected from the group consisting of cross-linked polyvinyl pyrrolidinone, cross-linked cellulose acetate phthalate, cross-linked hydroxypropyl methyl cellulose acetate succinate, microcrystalline cellulose, polyethylene/polyvinyl alcohol copolymer, polyethylene/polyvinyl pyrrolidinone copolymer, cross-linked carboxymethyl cellulose, sodium starch glycolat, and cross-linked styrene divinyl benzene, and/or wherein the activated carbon is selected from the group consisting of polyimides, polyarylonitrile, phenolic resins, cellulose acetate, regenerated cellulose, and rayon. Preferably, the substrate is selected from the group consisting of silicon dioxide, e.g. colloidal or fumed silicon dioxide or porous silica; copolymers, such as polyethylene/polyvinyl alcohol copolymer, polyethylene/polyvinyl pyrrolidinone copolymer; and cellulose, preferably microcrystalline cellulose.

Preferably, the dapagliflozin is stable in the amorphous state upon storage, optionally upon storage under stress conditions. A suitable test for determining stability is described elsewhere herein with respect to the solid dispersions of the invention.

In the adsorbate, said dapagliflozin can be associated with the substrate, and essentially the entire API, preferably all API, is present in amorphous form.

It is also preferred that the substrate has a high BET-surface area. A person skilled in the art knows what BET-surface area is "high", respectively based on the BET-surface areas the respective substrate can have. For instance, the BET-surface area is at least 1 $m^2/g$, preferably in a range of from 1 to 1000 $m^2/g$. The determination of the BET-surface area of the substrate can be carried out according to the method as described in the article: J. Am. Chem. Soc. 60, 309 (1938). Additionally, the substrates with the defined BET-surfaces can have a porosity as defined below. For instance, the porosity of the substrate can be at least 20 %, 30 %, 40 %, 50 % or 60 %. Also, the porosity can be in the range of between 10-70 %, or between 20-70 %, or between 30-70 % or between 40-70 %. The term "porosity" as used herein refers to the open pore porosity, which can be determined using the aforementioned method. The open pores of the substrate will typically be accessible to the solvent containing the API during the process for preparation of the adsorbates.

The obtained adsorbate according to the present invention can for instance be analyzed by SEM (magnification e.g. 100 times to 10000 times) or Raman imaging.

In a preferred embodiment, essentially all of the dapagliflozin is present in the adsorbate in amorphous form. The substrate according to the present invention may be a particulate and/or porous substrate, which means that this substrate has an outer and/or inner surface onto which the API can be adsorbed. Furthermore, the substrate according to the present invention does not essentially change its morphology during the adsorption of the API.

The porosity can be determined according to DIN EN 623-2, wherein the porosity can be at least 20 %, 30 %, 40 %, 50 % or 60 %. Also, the porosity can be in the range of between 10-70 %, or between 20-70 %, or between 30-70 %, or between 40-70 %.

Like in the solid dispersion, said dapagliflozin in the adsorbate is preferably present in its free form, in particular, dapagliflozin is not present in the form of solvates, hydrates or salts.

Preferably, the adsorbate does not contain crystalline portions, in particular no crystalline portions can be detected by X-ray powder diffraction measurement.

The amount of dapagliflozin in the adsorbate can be in the range of 0.01 to 40 wt.-%, preferably in the range of 0.1 to 30 wt.-%, more preferably in the range of 1 to 30 wt.-%, and even more preferably in the range of 1 to 20 wt.-% (respectively in % by weight relative to the whole adsorbate).

The invention also refers to a process for the preparation of an adsorbate described herein, comprising:

a) combining a solution of dapagliflozin of formula 1

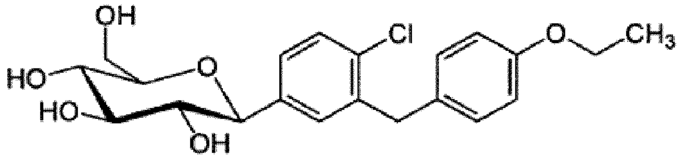

formula 1

in a solvent or mixture of solvents with a substrate, wherein the solvent or mixture of solvents is optionally selected from the group consisting of halogenated hydrocarbon, C5-C9 hydrocarbon, C1-C4 alcohol, C3-C6 ketone, organic ether, organic ester and mixtures thereof;
b) removing (or evaporating) the solvent or mixture of solvents under reduced pressure to form the adsorbate.

The solvent can be removed using any known methods. Preferably the solvent is removed by filtration or evaporation or by a combination of evaporation and filtration, more preferably the solvent is removed by evaporation. Also preferably the evaporation or evaporation and filtration is carried out in such a manner that the solvent is evaporated or evaporation and filtrated slowly, suitably during a period (evaporation period) of at least 30 minutes, further preferred at least 50 or 60 minutes. The maximum solvent removing time is 2 hours. Within the context of the present invention, the "evaporation period" corresponds to the time that is required to evaporate at least 80%, further preferred at least 90%, further preferred at least 95% of the solvent. In other words, the period of evaporation is determined by measuring the time during which e.g. at least 80% of the solvent is evaporated. Slowly removing the solvent has the benefit that uneconomic, complex and laborious process steps, which would otherwise be necessary to achieve a fast removal, are not necessary. Additionally, the slow removal of the solvent leads to the formation of a stable adsorbate having improved properties e.g. with respect to stability and solubility of the API. That is the process disclosed allows equilibrated formation of associative forces between the API substance and the surface of the substrate material to provide enhanced API stability, while later, when put in aqueous solution, it allows to achieve enhanced and relatively fast dissociation of the afore-mentioned forces. If the evaporation/removing of the solvent is carried out too fast, the formation of e.g. coprecipitates of the API occurs, which would not be adsorbed on the substrate.

The invention also refers to a process for the preparation of a pharmaceutical composition comprising an adsorbate described herein, comprising:

a) providing a mixture of the adsorbate, wherein the adsorbate has been prepared as described elsewhere herein, and at least one pharmaceutically acceptable excipient;
b) optionally fine-milling and/or sieving the mixture obtained in step a);
c) formulation of the mixture of step a) or b) into a pharmaceutical composition by dry formulation.

Fine-milling and sieving are methods that are well known to a person skilled in the art. Any suitable fine-milling or sieving method can be used.

The same is true for the dry formulation of the mixture into a pharmaceutical composition.

The invention also refers to a pharmaceutical composition comprising the adsorbate described herein or obtainable by the process described herein, and one or more pharmaceutically excipients e.g. selected from the group consisting of fillers, disintegrants, binders, lubricants, and surfactants.
The amount of the adsorbate in the pharmaceutical composition can be in the range of 1 to 95 wt.-%, preferably in the range of 5 to 90 wt.-%, more preferably in the range of 10 to 70 wt.-% and even more preferably in the range of 20 to 50 wt.-% (respectively in % by weight relative to the whole pharmaceutical composition).

The pharmaceutical composition according to the present invention, and preferably the immediate release tablet of the present invention, comprises at least one excipient. Generally, there are no specific restrictions concerning the chemical nature of these excipients provided that the excipient or mixture of excipients comprised in the oral solid dosage form is/are pharmaceutically acceptable. A pharmaceutically acceptable excipient is an excipient which is relatively non-toxic and innocuous to a patient at concentrations consistent with effective activity of the amorphous dapagliflozin so that any side effects ascribable to the excipient do not vitiate the beneficial effects of the amorphous dapagliflozin. Therefore, according to the present invention, excipients are, for example, disintegrants, binders, lubricants, fillers, plasticizers, surfactants and wetting agents, film-forming agents and coating materials, sweeteners, flavoring agents, and coloring agents such as example pigments. Other excipients known in the field of pharmaceutical compositions may also be used.

The fillers may be selected from the group consisting of different grades of starches, such as maize starch, potato starch, rice starch, wheat starch, pregelatinized starch, fully pregelatinized starch; cellulose derivatives, such as microcrystalline cellulose or silicified microcrystalline cellulose; sugar alcohols such as mannitol, erythritol, sorbitol, xylitol; monosaccharides like glucose; oligosaccharides like sucrose and lactose such as lactose monohydrate, lactose anhydrous, spray dried lactose or anhydrous lactose; calcium salts, such as calcium hydrogenphosphate; particularly preferably the fillers are selected from the group consisting of, microcrystalline cellulose, silicified microcrystalline cellulose, lactose monohydrate, spray dried lactose, and anhydrous lactose.

The disintegrants may be selected from the group consisting of carmellose calcium, carboxymethylstarch sodium, croscarmellose sodium (cellulose carboxymethylether sodium salt, crosslinked), starch, modified starch such as pregelatinized starch, starch derivatives such as sodium starch glycolate, crosslinked polyvinylpyrrolidone (crospovidone), and low-substituted hydroxypropylcellulose, and disintegrating aids such as magnesium slumino-metasilicate and ion exchange resins like polacrilin potassium; particularly preferably the disintegrants are selected from the group consisting of sodium starch glycolate, croscarmellose sodium and crospovidone.

The lubricants may be selected from the group consisting of stearic acid, talc, glyceryl behenate, sodium stearyl fumarate and magnesium stearate; particularly preferably the lubricant are magnesium stearate and sodium stearyl fumarate.

The binders may be selected from the group consisting of polyvinyl pyrrolidone (Povidone), polyvinyl alcohol, copolymers of vinylpyrrolidone with other vinylderivatives (Copovidone), hydroxypropyl methylcellulose, methylcellulose, hydroxypropylcellulose, powdered acacia, gelatin, guar gum, carbomer such as carbopol, polymethacrylates and pregelatinized starch.

Diluents may correspond to the fillers listed above.

Glidants may be selected from the group consisting of colloidal silica, hydrophobic colloidal silica and magnesium trisilicate, such as talc; particularly preferably the glidants are selected from the group consisting of colloidal silica and hydrophobic colloidal silica.

Suitable sweeteners may be selected from the group consisting of aspartame, saccharin sodium, dipotassium glycyrrhizinate, aspartame, stevia, thaumatin, and the like.

Preferably the excipients are microcrystalline cellulose, silicified microcrystalline cellulose, anhydrous lactose, lactose monohydrate, spray dried lactose, croscarmellose sodium, sodium starch glycolate, low substituted hydroxypropylcellulose, crospovidone, magnesium stearate, and sodium stearyl fumarate.

Suitable film-forming agents and coating materials according to the present invention include, but are not limited to, , hydroxypropyl methylcellulose (hypromellose, HPMC), hydroxypropyl cellulose, polyvinylalcohol, , methylcellulose, ethylcellulose, hydroxypropylmethyl cellulose phthalate, hydroxypropylmethyl cellulose acetate succinate, shellac, liquid glucose, hydroxyethyl cellulose, polyvinylpyrrolidone, copolymers of vinylpyrrolidone and vinylacetate such as Kollidon® VA64 BASF, copolymers of acrylic and/or methacrylic acid esters with trimethylammoniummethylacrylate, copolymers of dimethylaminomethacrylic acid and neutral methacrylic acid esters, polymers of methacrylic acid or methacrylic acid esters, copolymers of acrylic acid ethylester and methacrylic acid methyl ester, and copolymers of acrylic acid and acrylic acid methylester.

Suitable plasticizers according to the present invention include, but are not limited to, polyethylene glycol, diethyl phthalate and glycerol. Preference is given to polyethylene glycol. Suitable coloring agents according to the present invention include, but are not limited to, pigments, inorganic pigments, FD&C Red No. 3, FD&C Red No. 20, FD&C Yellow No. 6, FD&C Blue No. 2, D&C Green No. 5, D&C Orange No. 5, D&C Red No. 8, caramel, ferric oxide red, ferric oxide yellow and titanium dioxide.

Suitable further commonly used excipients which may be used according to the present invention include, but are not limited to, acidifying agents such as acetic acid, citric acid, fumaric acid, hydrochloric acid and nitric acid; alkalizing agents such as ammonia solution, ammonium carbonate, diethanolamine, monoethanolamine, potassium hydroxide, sodium borate, sodium carbonate, sodium hydroxide, triethanolamine and trolamine; adsorbents such as powdered cellulose and activated charcoal; stabilizers and antioxidants such as ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate and sodium metabisulfite; binding materials such as block polymers, natural and synthetic rubber, polyacrylates, polyurethanes, silicones, polysiloxanes and styrene-butadiene copolymers; buffering agents such as potassium metaphosphate, dipotassium phosphate, sodium acetate, sodium citrate anhydrous and sodium citrate hydrates; encapsulating agents such as gelatin, starch and cellulose derivates; flavorants, masking agents and odors such as anise oil, cinnamon oil, cocoa, menthol, orange oil, peppermint oil and vanillin; humectants such as glycerol, propylene glycol and sorbitol; sweeteners such as aspartame, dextrose, glycerol, mannitol, propylene glycol, saccharin sodium, sorbitol and sucrose; anti-adherents such as magnesium stearate and talc; direct compression excipients such as dibasic calcium phosphate, lactose and microcrystalline cellulose; tablet polishing agents such as carnauba wax and white wax.

The skilled person will appreciate that depending upon formulation context and concentration a particular

excipient can fulfill various and sometimes even different functions. For example, microcrystalline cellulose is a particular hydrolyzed cellulose, which can be used as a filler, binder and/or disintegrating material in tablet production, dependent on formulation context and concentration. Reference is made to the literature on pharmaceutical excipients and pharmaceutical formulation, such as Fiedler Encyclopedia of Excipients for Pharmaceuticals, Cosmetics and Related Areas. Wissenschaftliche Verlagsgesellschaft Stuttgart, 2013, Bauer, Frömming and Führer, "Lehrbuch der Pharmazeutischen Technologie" Wissenschaftliche Verlagsgesellschaft Stuttgart, 9. Auflage (2012) or, with a particular focus on tablet production, Augsburger and Stephen, Pharmaceutical Dosage Forms: Tablets, Third Edition, Volume 2, Informa Healthcare (2008). The skilled person will therefore appreciate that terms like "disintegrant", "binder", "lubricant", "filler", "plasticizer", "surfactant", "wetting agent", "film-forming agent", "coating material", "sweetener", "flavoring agent" and "coloring agent" are primarily functional definitions and that the structural characterization provided above are given so as to more easily allow identification of suitable excipients.

The oral solid dosage form of the present invention is preferably a compressed or a non-compressed dosage form. Preferably, the oral solid dosage form of the present invention is a granule, a capsule, for example a capsule filled with granules, a sachet, a pellet, a dragee, a lozenge, a troche, a pastille, or a tablet, such as an uncoated tablet, a coated tablet, an effervescent tablet, a soluble tablet, a dispersible tablet, an orodispersible tablet, a tablet for use in the mouth, a chewable tablet or an extrudate. Preferably, the longest dimension of an oval or round tablet or of a capsule is at most about 35 mm.

According to a preferred embodiment of the present invention, the pharmaceutical composition is a compressed dosage form. More preferably, the pharmaceutical composition is a tablet. Tablets can be prepared by compressing uniform volumes of particles or particle aggregates, preferably produced by granulation methods. In the manufacture of such tablets, means are taken to ensure that they possess a suitable mechanical strength to avoid crumbling or breaking on handling or subsequent processing. The process of providing tablets is well known to the skilled person. Most preferably, the pharmaceutical composition is an immediate release tablet. Also most preferably, dapagliflozin is present in the pharmaceutical composition in pure amorphous form.

According to this embodiment, the dosage form, preferably tablet, of the present invention preferably comprises at least one excipient selected from the group consisting of fillers, disintegrants, lubricants, and surfactants. More preferably, the dosage form of the present invention comprises at least one filler and at least one disintegrant and at least one lubricant and at least one surfactant. Even more preferably, the tablet comprises at least one filler, preferably microcrystalline cellulose in an amount of from 40% to 95% by weight, at least one disintegrant, preferably croscarmellose sodium, in an amount of from 2% to 10% by weight, at least one binder, preferably Povidone, at least one lubricant, preferably magnesium stearate, in an amount of from 0.5% to 5% by weight, and at least one surfactant, such as NaLS, in each case relative to the total weight of the tablet.

The tablet of the present invention usually contains about 1- about 10% by weight of the dapagliflozin based on the total weight of the pharmaceutical composition. More preferably, the respective content is 4- about 8%.

A preferred dissolution rate of the tablet according to this embodiment of the present invention is at least 50% in 15min. Preferably, the dissolution rate is at least 90 % in 50min, more preferably at least 50% in 15min and at least 90% in 50min, and in particular at least 80% in 15min and at least 90% in 40min.

According to an especially preferred embodiment, the present invention relates to a tablet as specified in Example 1, 2, 3 or 4. As to this especially preferred embodiment of the present invention, it was found that said tablets for instance exhibit improved content uniformity and/or improved dissolution properties compared to prior art tablets.

The pharmaceutical composition can be administered to patients in a country having an area with an Af or an Am climate, preferably an Af climate, according to the Köppen-Geiger climate classification.

The Köppen-Geiger classification is one of the most widely used climate classification systems. It combines average annual and monthly temperatures and precipitation, and the seasonality of precipitation in an area. Examples of countries having an area with an Af climate are Brazil, Indonesia, Mexico, Puerto Rico, Zaire, to name but a few. Examples of countries having an area with an Am climate are Brazil, Indonesia, Mexico, Cuba, the USA, Zaire, India, China, Birma, to name but a few.

Therefore, the present invention also relates to the pharmaceutical composition, e.g. the oral solid dosage form, e.g. the tablet, of the present invention for use in the treatment of type II diabetes, wherein said pharmaceutical composition, e.g. the oral dosage form, e.g. the tablet, is to be administered to patients in a country having an area with an Af or an Am climate, preferably an Af climate, according to the Köppen-Geiger climate classification. Moreover the present invention relates to the pharmaceutical composition, e.g. the oral solid dosage form, e.g. the tablet, of the present invention for use in the treatment of type II diabetes, wherein the pharmaceutical composition, e.g. the oral dosage form, e.g. the tablet, is to be administered to patients in a country having an area with an Af or an Am climate, preferably an Af climate, according to the Köppen-Geiger climate classification, and wherein the pharmaceutical composition, e.g. the oral dosage form, is packaged in a packaging material having a vapour transmission rate of at least 0.4 g $m^{-2}$ $d^{-1}$, preferably of at least 1 g $m^{-2}$ $d^{-1}$, more preferably of at least 2 g $m^{-2}$ $d^{-1}$, as measured according to standard DIN 53122-1.

The present invention also relates to amorphous dapagliflozin for use in the treatment of diseases related to

hypoglycemia, such as type II diabetes, wherein the pharmaceutical composition, e.g. the oral dosage form, e.g. the tablet, optionally is to be administered to patients in a country having an area with an Af or an Am climate, preferably an Af climate, according to the Köppen-Geiger climate classification. The present invention also relates to amorphous dapagliflozin for the preparation of a pharmaceutical composition, e.g. dosage form, which pharmaceutical composition, e.g. dosage form, is packaged in a packaging material having a vapour transmission rate of at least 0.4 g $m^{-2}$ $d^{-1}$, preferably of at least 1 g $m^{-2}$ $d^{-1}$, more preferably of at least 2 g $m^{-2}$ $d^{-1}$, as measured according to standard DIN 53122-1. Preferred dosage forms in this embodiment are oral solid dosage form, e.g. tablets.

Further, the present invention relates to the use of amorphous dapagliflozin for the preparation of a pharmaceutical composition, e.g. an oral solid dosage form, e.g. a tablet, having increased chemical stability after packaging in a polypropylene film and storage in the dark at 40 °C at a relative humidity of 75 % for a period of at least 14 days, compared to an identically packaged and stored pharmaceutically composition e.g. oral solid dosage form comprising, instead of dapagliflozin S-propylene glycol hydrate, amorphous dapagliflozin.

Following from this, the pharmaceutical composition can be packaged in a packaging material having a moisture vapour transmission rate of at least 0.4 g $m^{-2}$ $d^{-1}$ as measured according to standard DIN 53122-1, said packaging material preferably being made from polypropylene, polyvinylidenchloride and/or polyvinylchloride.

Further, the invention also refers to a solid dispersion, adsorbate, granule, or pharmaceutical composition described herein for use in the treatment of type II diabetes mellitus, optionally in patients in a country having an area with an Af or an Am climate according to the Köppen-Geiger climate classification.

## Description of the figures

**Figure 1** shows the dissolution of dapagliflozin from 5 mg tablets.

**Figure 2** shows the dissolution of dapagliflozin from 5 mg tablets - variability between results set.

**Figure 3** shows an SEM-image (2000 x magnification) of microcrystalline cellulose Avicel PH101

**Figure 4** shows an SEM-image (2000 x magnification) of an adsorbate on microcrystalline cellulose Avicel PH101, wherein no particles of dapagliflozin can be observed. BET measurement shows significant decrease in specific surface area: 0,12 $m^2$/g (adsorbate) compared to 1,1 $m^2$/g (pure MCC). This indicates that the surface layer of MCC could be loaded with dapagliflozin and its porosity and specific surface area decreased consequently.

**Figure 5** shows an SEM-image (2000 x magnification) of silicon dioxide Syloid Al-1.

**Figure 6** shows an SEM-image (2000 x magnification) of an adsorbate on silicon dioxide Syloid Al-1, wherein no particles of dapagliflozin can be observed. BET measurement shows significant decrease in specific surface area: 24 $m^2$/g compared to 750 $m^2$/g (pure Syloid). This indicates that the pores of Syloid could be loaded and filled with dapagliflozin and specific surface area decreased consequently.

Further, XRP diffractograms of the tablets according to the present invention only show placebo peaks, thus confirming that only amorphous dapagliflozin is present in the tablet samples.

## Examples

Amorphous dapagliflozin was provided according to the methods described in WO 2004/063209.

Table 1: Equilibrium solubility of dapagliflozin and dapagliflozin propylene glycol solvate hydrate (24 hours at 37°C)

| Medium | Solubility (mg/mL) | | Desciptive term | pH of suspension after 24 hours of testing | |
|---|---|---|---|---|---|
| | amorphous dapagliflozine | dapaglifloz in propylene glycol solvate hydrate | | amorphous dapagliflozine | dapaqliflozin propylene glycol solvate hydrate |
| pH 1.2 | 1.2 | 1.7 | Slightly soluble | 1,2 | 1,3 |
| pH 3.0 | 1.1 | / | Slightly soluble | 2,9 | / |
| pH 4.5 | 1.2 | / | Slightly soluble | 4,5 | / |

(continued)

| Medium | Solubility (mg/mL) | | Desciptive term | pH of suspension after 24 hours of testing | |
|---|---|---|---|---|---|
| | amorphous dapagliflozine | dapaglifloz in propylene glycol solvate hydrate | | amorphous dapagliflozine | dapaqliflozin propylene glycol solvate hydrate |
| pH 6.8 | 1.2 | 1.7 | Slightly soluble | 6,7 | 6,8 |
| pH 7.4 | 0.7 | / | Very slightly soluble | 7,2 | / |
| water | 1.2 | 1.8 | Slightly soluble | 7,4 | 3,2 |

The above table provides a comparison of the properties of dapagliflozin and dapagliflozin propylene glycol solvate hydrate. As can be derived from said table, dapagliflozin propylene glycol solvate hydrate has superior dissolution properties and thus seems to represent the more suitable form of dapagliflozin for formulating dosage forms. In other words, providing dosage forms containing amorphous dapagliflozin and having a very good dissolution profile is more demanding.

Furthermore, pure amorphous dapagliflozin has a low glass transition temperature (around 40°C). Further, it is hygroscopic, which means that it absorbs around 6% of water at 80% relative air humidity (RH). The sorption and desorption during the first cycle exhibit hysteresis. Desorption is incomplete, and around 3% of water is irreversibly absorbed during the first cycle, which results in the change of consistence from powder to semisolid. These properties, i.e. the low glass transition temperature and hygroscopicity, have a negative impact on processibility of dapagliflozin, going along with a negative influence on important properties of pharmaceutical compositions comprising dapagliflozin which contribute to the desired therapeutic effect, such as content uniformity, storage stability or dissolution profile. Other forms of dapagliflozin, e.g. dapagliflozin propylene glycol hydrate is only slightly hygroscopic, as it absorbs only around 0.8% of water at 80% RH.

Example 1:

Tablet composition:

| Substance | Amount per tablet (mg) |
|---|---|
| Dapagliflozin | 5.00 |
| Microcrystalline cellulose | 114.20 |
| Hypromellose | 3.00 |
| Croscarmellose sodium | 6.50 |
| Magnesium stearate | 1.30 |
| Total mass (mg) | 130.00 |

Manufacturing procedure:

Dapagliflozin is dissolved in a suitable amount of ethanol. Microcrystalline cellulose, hypromellose and croscarmellose sodium are pre-mixed in a high shear granulator. Dapagliflozin solution is sprayed onto said powder mixture during mixing to obtain wet granulate. The granulate is dried in a fluid bed processor, sieved blended with magnesium stearate and compressed into tablets.

Example 2:

Tablet composition:

| Substance | Amount per tablet (mg) |
|---|---|
| Dapagliflozin | 5.00 |
| Polyvinyl alcohol | 10.00 |
| Microcrystalline cellulose | 103.00 |
| Croscarmellose sodium | 10.00 |
| Magnesium stearate | 2.00 |
| Total mass (mg) | 130.00 |

Manufacturing procedure:

Polyvinyl alcohol is dissolved in a suitable amount of ethanol/acetone/water. Dapagliflozin is dissolved in polyvinyl alcohol ethanol solution. The prepared solution is sprayed onto microcrystalline cellulose and croscarmellose sodium in a fluid bed apparatus. After spraying is completed, the powder is dried and sieved. The obtained powder containing a solid dispersion is blended with magnesium stearate and compressed into tablets.

Example 3:

Tablet composition:

| Substance | Amount per tablet (mg) |
|---|---|
| Dapagliflozin | 5.00 |
| Povidone | 10.00 |
| Microcrystalline cellulose | 103.00 |
| Croscarmellose sodium | 10.00 |
| Magnesium stearate | 2.00 |
| Total mass (mg) | 130.00 |

Manufacturing procedure:

Povidone is dissolved in a suitable amount of ethanol/acetone/water. Dapagliflozin is dissolved in said povidone solution. The prepared solution is sprayed onto microcrystalline cellulose and croscarmellose sodium in fluid bed apparatus. After spraying is completed powder is dried and sieved. Obtained powder containing solid dispersion is blended with magnesium stearate and compressed into tablets.

Example 4:

Adsorbate Composition:

| Substance | Amount per tablet (mg) |
|---|---|
| Dapagliflozin | 5.00 |
| Microcrystalline cellulose | 133.00 |
| Total mass (mg) | 138.00 |

Manufacturing procedure:

5 g of dapagliflozin was dissolved in a mixture of 200 ml tert-butyl methyl ether, 400 ml chloroform and 400 ml of hexane. 532 g of Avicel PH 101 was added to the solution and stirred. The solvents were slowly removed under reduced pressure over a period of one hour. The solvents were further removed at 50 °C and 10 mbar for 8 hours.

Tablet composition:

| Substance | Amount per tablet (mg) |
|---|---|
| Dapagliflozin adsorbate | 138.00 |
| Microcrystalline cellulose | 50.00 |
| Croscarmellose sodium | 10.00 |
| Magnesium stearate | 2.00 |
| Total mass (mg) | 200.00 |

Manufacturing procedure:

Dapagliflozin adsorbate, microcrystalline cellulose, croscarmellose sodium are blended and sieved. Magnesium stearate is added and the mixture is blended.
Prepared final blend is compressed into tablets.

Comparative Example 1:

Tablet composition:

| Substance | Amount per tablet (mg) |
|---|---|
| Dapagliflozin | 5.00 |
| Microcrystalline cellulose | 117.20 |
| Croscarmellose sodium | 6.50 |
| Magnesium stearate | 1.30 |
| Total mass (mg) | 130.00 |

Manufacturing procedure:

Dapagliflozin, microcrystalline cellulose, croscarmellose sodium are blended and sieved. Magnesium stearate is added and the mixture is blended.
Prepared final blend is compressed into tablets.

Comparative Example 2:

Tablet composition:

| Substance | Amount per tablet (mg) |
|---|---|
| Dapagliflozin | 5.00 |
| Microcrystalline cellulose | 114.20 |
| Hypromellose | 3.00 |
| Croscarmellose sodium | 6.50 |
| Magnesium stearate | 1.30 |

(continued)

| Substance | Amount per tablet (mg) |
|---|---|
| Total mass (mg) | 130.00 |

Manufacturing procedure:

Dapagliflozin, microcrystalline cellulose, hypromellose and croscarmellose sodium are pre-mixed in a high shear granulator. Ethanol is sprayed onto powder mixture during mixing to obtain a wet granulate.
The granulate is dried in a fluid bed processor, sieved blended with magnesium stearate and compressed into tablets.

Comparative Example 3:

Tablet composition:

| Substance | Amount per tablet (mg) |
|---|---|
| Dapagliflozin | 5.00 |
| Microcrystalline cellulose | 88.500 |
| Lactose monohydrate | 25.000 |
| Cross povidone | 7.500 |
| Colloidal Anhydrous Silica | 2.500 |
| Magnesium stearate | 1.50 |
| Total mass (mg) | 130.00 |

Manufacturing procedure:

Dapagliflozin, microcrystalline cellulose, lactose monohydrate, crospovidone, and colloidal anhydrous silica are blended and sieved. Magnesium stearate is added and the mixture is blended. The prepared final blend is compressed into tablets.

Table 2: Dapagliflozin Assay determination in individual tablet (Content Uniformity)

| | **EXAMPLE 1** | **EXAMPLE 2** | **EXAMPLE 3** | **EXAMPLE 4** | **COMPARATIVE EXAMPLE 1** | **COMPARATIVE EXAMPLE 2** |
|---|---|---|---|---|---|---|
| Tablet 1 | 96.7 | 104.7 | 105.7 | 98.5 | 112.4 | 101.7 |
| Tablet 2 | 95.6 | 102.5 | 107.2 | 98.9 | 108.1 | 89.9 |
| Tablet 3 | 96.4 | 104.8 | 108.7 | 97.5 | 99.8 | 84.2 |
| Tablet 4 | 96.1 | 104.9 | 106.0 | 96.0 | 97.7 | 94.1 |
| Tablet 5 | 96.9 | 102.7 | 106.2 | 96.6 | 93.0 | 92.5 |
| Tablet 6 | 96.8 | 104.7 | 105.5 | 99.0 | 110.1 | 87.3 |
| Tablet 7 | 100.2 | 103.8 | 108.1 | 99.4 | 106.5 | 86.8 |
| Tablet 8 | 97.3 | 105.0 | 107.9 | 98.7 | 92.4 | 84.0 |
| Tablet 9 | 95.8 | 103.4 | 105.7 | 98.6 | 109.4 | 89.0 |
| Tablet 10 | 98.0 | 103.6 | 104.9 | 101.5 | 113.6 | 88.2 |
| **Average** | **97.0** | **104.0** | **106.6** | **98.5** | **104.3** | **89.8** |
| **RSD** | **1.3** | **0.9** | **1.2** | **1.6** | **7.6** | **5.9** |

(continued)

| | EXAMPLE 1 | EXAMPLE 2 | EXAMPLE 3 | EXAMPLE 4 | COMPARATIVE EXAMPLE 1 | COMPARATIVE EXAMPLE 2 |
|---|---|---|---|---|---|---|
| **AV[1]** | **4.6** | **4.8** | **8.2** | **3.7** | **21.9** | **21.4** |

[1]Acceptance value (AV) is a generally applied calculated measure for evaluation of content uniformity. General calculation of AV is based on a composite criteria of mean content difference from targeted value (most commonly 100%) and variability on the assay of the individual contents (presented as standard deviation), and is calculated using the following formula:

$$| M - X | + kS$$

Where,

M = Reference value, depends on targeted value and mean of individual contents

X = Mean of individual contents

k = Acceptability constant, (If n = 10 then k = 2.4,

If n = 30, then k = 2.0)

S = Sample standard deviation.

An acceptance value (AV) above 10 (preferably above 15) is considered to be an indicative of inacceptable content uniformity. As presented in Table 2, examples 1-4 showed superior content uniformity (calculated as shown above) compared to comparative examples 1 and 2.

Table 3: Dapagliflozin stability

| | | EXAMPLE 1 | EXAMPLE 2 | EXAMPLE 3 | EXAMPLE 4 | COMPARATIVE EXAMPLE 1 | COMPARATIVE EXAMPLE 2 |
|---|---|---|---|---|---|---|---|
| **INITIAL** | Highest individual | <0.05 | <0.05 | <0.05 | <0.05 | 0.05 | <0.05 |
| | **Sum of impurities** | **<0.05** | **<0.05** | **<0.05** | **<0.05** | **0.05** | **<0.05** |
| **40°C/65% RH 21 days** | Highest individual | <0.05 | <0.05 | <0.05 | 0.05 | <0.05 | 0.07 |
| | **Sum of impurities** | **<0.05** | **<0.05** | **<0.05** | **0.05** | **<0.05** | **0.05** |
| **40°C 1 month** | Highest individual | <0.05 | <0.05 | <0.05 | 0.08 | 0.05 | 0.07 |
| | **Sum of impurities** | **<0.05** | **<0.05** | **<0.05** | **0.08** | **0.05** | **0.12** |
| **40°C 3 months** | Highest individual | 0.07 | 0.05 | <0.05 | 0.09 | not tested | 0.09 |
| | **Sum of impurities** | **0.07** | **0.05** | **<0.05** | **0.09** | **not tested** | **0.09** |

Results from stability testing are presented in Table 3. Samples were exposed to storage on stress and accelerated conditions, and afterwards analysed with HPLC method. As can be derived from the Table 3, the samples described in examples 1-4 remain stable under storage on stress and accelerated conditions, in particular no observed increased degradation trends or smaller, compared to comparative examples 1 and 2.

Dissolution testing for dapagliflozin tablets was performed with following parameters: 500 ml of dissolution medium 0.1 M HCl, Apparatus 2 at 50 rpm, peak vessel, at 37°C. Some tests were also made in phosphate buffer at pH 6.8.

Dissolution profiles of examples 1, 2, and 3 as well as comparative examples 1, 2, 3 and 4 are presented in Figure 1. Fast and complete dissolution of dapagliflozin was obtained for example 1, 2, 3 and 4 where most of the substance is dissolved within the first 5 minutes. Dissolution of dapagliflozin of comparative examples 1 and 3 is slower and complete release of dapagliflozin is reached in 30 to 45 minutes. Comparative example 2 does not reach complete release of dapagliflozin from tablet when stirring is at 50 rpm. An increase of rotation to 150 rpm for 5 minutes at the end of the test is needed in order to complete the release from comparative example 2.

From the dissolution profiles presented in Figure 1 it can be concluded that examples 1, 2, 3 and 4 have superior dissolution in comparison to comparative examples 1, 2 and 3. Dissolution of comparative example 2 was found to be inappropriate.

In Figure 2 three sets of dissolution profiles obtained from comparative example 2 are presented. Unexpectedly low profile of comparative example 2 was repeated and two more results sets were obtained. Huge variability between the result sets was detected. Changes in dissolution profile are unacceptable since release of substance from tablet dictates the in vivo performance. This variability in dissolution results was not detected for other samples (example 1, 2, 3, 4 and comparative example 1 and 3).

**Claims**

1. Amorphous solid dispersion of at least one suitable polymer and dapagliflozin ((2S,3R,4R,5S,6R)-2-[4-chloro-3-(4-ethoxybenzyl)phenyl]-6-(hydroxymethyl)-tetrahydro-2H-pyran-3,4,5-triol) of formula 1

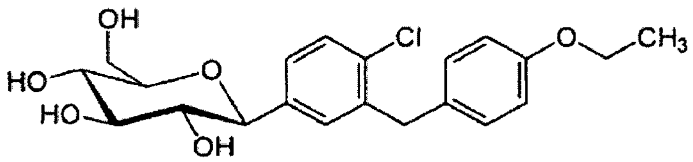

formula 1.

wherein the at least one polymer is selected from the group consisting of polyvinyl pyrrolidone (PVP), polyvinyl alcohol (PVA), polyacrylic acid (PAA), poly(ethylene glycol) (PEG), poly(ethylene oxide) (PEO), hydroxypropyl cellulose (HPC), hydroxypropyl methyl cellulose (HPMC), copovidone, hypromellose acetate succinate (AQOAT), polyacrylates, and mixtures thereof,
and wherein dapagliflozin is present in its free form.

2. The solid dispersion according to claim 1, wherein the weight ratio of dapagliflozin and the at least one polymer is from 1:10 to 10:1, preferably 1:1 to 1:10.

3. Pharmaceutical composition comprising the solid dispersion according to claim 1 or 2 and one or more pharmaceutically excipients selected from the group consisting of fillers, disintegrants, binders, lubricants, and surfactants, wherein the pharmaceutical composition comprises dapagliflozin as the sole pharmaceutically active ingredient.

4. Process for the preparation of the solid dispersion according claim 1 or 2, or a pharmaceutical composition according to claim 3, comprising:

a) providing a solution of dapagliflozin and at least one suitable polymer in a suitable solvent or mixture of solvents;
b) optionally spraying or dispersing the solution of step (a) onto a carrier to form granules.
c) evaporating the solvent, wherein the evaporation step is preferably carried out by fluid bed drying, spray drying, freeze drying (lyophilisation), vacuum drying, tray drying, microwave drying or other processes that result in solvent evaporation, thereby resulting in the formation of solid dispersion; and
d) optionally blending the obtained solid dispersion of steps b) or c) with one or more pharmaceutically acceptable excipients.

5. Process for the preparation of a pharmaceutical composition, wherein dapagliflozin is present in the pharmaceutical composition only as amorphous dapagliflozin, comprising:

a) providing a solution of dapagliflozin of formula 1

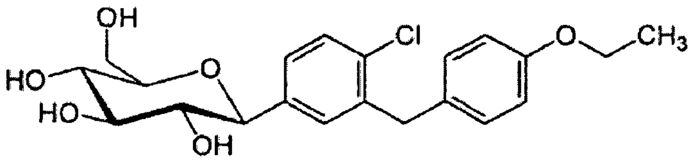

formula 1

and optionally at least one suitable polymer in a suitable solvent or mixture of solvents;
b) optionally spraying or dispersing the solution of step (a) onto carrier particles to form granules;
c) evaporating the solvent; and
d) blending the obtained composition of steps b) or c) with one or more pharmaceutically acceptable excipients.

6. Solid dispersion or pharmaceutical composition obtainable by the process according to claim 4 or 5.

7. Adsorbate comprising dapagliflozin ((2S,3R,4R,5S,6R)-2-[4-chloro-3-(4-ethoxybenzyl)phenyl]-6-(hydroxymethyl)-tetrahydro-2H-pyran-3,4,5-triol) of formula 1 adsorbed onto the surface of a substrate

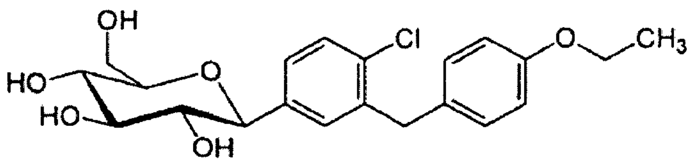

formula 1,

wherein dapagliflozin is substantially amorphous and wherein the substrate is selected from the group consisting of

(a) an inorganic oxide;
(b) water insoluble inorganic salt;
(c) water insoluble polymer; and
(d) an activated carbon,

wherein dapagliflozin is not present in the form of particles.

8. The adsorbate according to claim 7, wherein the substrate is selected from the group consisting of silicon dioxide and microcrystalline cellulose.

9. Process for the preparation of an adsorbate according to claim 7 or 8, comprising:

a) combining a solution of dapagliflozin of formula 1

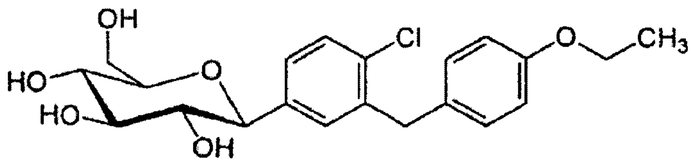

formula 1

in a solvent or mixture of solvents with a substrate;
b) removing the solvent or mixture of solvents under reduced pressure to form the adsorbate.

10. Process for the preparation of a pharmaceutical composition comprising an adsorbate as defined in claim 7 or 8, comprising:

a) providing a mixture of the adsorbate, and at least one pharmaceutically acceptable excipient;
b) optionally fine-milling and/or sieving the mixture obtained in step a);
c) formulation of the mixture of step a) or b) into a pharmaceutical composition by dry formulation.

**11.** Pharmaceutical composition comprising the adsorbate as defined in claim 7 or 8 or obtainable by the process according to claim 10, and one or more pharmaceutically excipients selected from the group consisting of fillers, disintegrants, binders, lubricants, and surfactants.

**12.** The pharmaceutical composition according to any one of the preceding claims, wherein the pharmaceutical composition is a compressed dosage form, preferably a tablet, more preferably an immediate release tablet, and/or wherein dapagliflozin is only present in the pharmaceutical composition as amorphous dapagliflozin.

**13.** The pharmaceutical composition according to any of the preceding claims, wherein the content uniformity, indicated in terms of acceptance value (AV) as defined by the legend of Table 2, of said pharmaceutical composition being packaged in a packaging material such as a blister containing N tablets is below 15, preferably below 10.

**14.** The pharmaceutical composition according to any of the preceding claims, wherein, when carrying out dissolution testing, at a time point of 5 minutes more than 80%, preferably more than 85% of the dapagliflozin is dissolved, wherein the dissolution testing is carried out by applying the following parameters: 500 ml of dissolution medium 0.1 M HCl, Apparatus 2 at 50 rpm, peak vessel, 37°C.

**15.** Solid dispersion, adsorbate or pharmaceutical composition according to any one of the preceding claims for use in the treatment of diseases related to hypoglycemia.

**Patentansprüche**

**1.** Amorphe feste Dispersion aus mindestens einem geeigneten Polymer und Dapagliflozin ((2S, 3R, 4R, 5S, 6R)-2-[4-Chlor-3-(4-ethoxybenzyl)phenyl]-6-(hydroxymethyl)-tetrahydro-2H-pyran-3,4,5-triol) der Formel 1

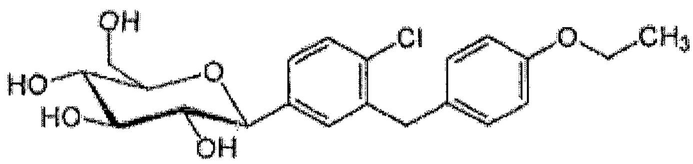

Formel 1

wobei das mindestens eine Polymer ausgewählt ist aus der Gruppe bestehend aus Polyvinylpyrrolidon (PVP), Polyvinylalkohol (PVA), Polyacrylsäure (PAA), Poly(ethylenglykol) (PEG), Poly(ethylenoxid) (PEO), Hydroxypropylcellulose (HPC), Hydroxypropylmethylcellulose (HPMC), Copovidon, Hypromelloseacetatsuccinat (AQOAT), Polyacrylate, und Mischungen davon,
und wobei Dapagliflozin in seiner freien Form vorliegt.

**2.** Feste Dispersion gemäß Anspruch 1, wobei das Gewichtsverhältnis von Dapagliflozin und dem mindestens einen Polymer von 1:10 bis 10:1, bevorzugt 1:1 bis 1:10, beträgt.

**3.** Pharmazeutische Zusammensetzung umfassend die feste Dispersion gemäß Anspruch 1 oder 2 und einen oder mehrere pharmazeutische Hilfsstoffe ausgewählt aus der Gruppe bestehend aus Füllstoffen, Sprengmitteln, Bindemitteln, Gleitmitteln und Tensiden, wobei die pharmazeutische Zusammensetzung Dapagliflozin als den einzigen pharmazeutisch aktiven Wirkstoff umfasst.

**4.** Verfahren zur Herstellung der festen Dispersion gemäß Anspruch 1 oder 2, oder einer pharmazeutischen Zusammensetzung gemäß Anspruch 3, umfassend:

a) Bereitstellen einer Lösung von Dapagliflozin und mindestens einem geeigneten Polymer in einem geeigneten Lösungsmittel oder Mischung von Lösungsmitteln;
b) optional, Sprühen oder Dispergieren der Lösung von Schritt (a) auf einen Träger, um Granulen zu bilden.
c) Evaporieren des Lösungsmittels, wobei der Evaporierungsschritt bevorzugt ausgeführt wird durch Flüssigbetttrocknen, Sprühtrocknen, Gefriertrocknen (Lyophilisation), Vakuumtrocknen, Schalentrocknen, Mikrowellentrocknen oder andere Verfahren, die in Lösungsmittel-Evaporation resultieren, dadurch resultierend in der Bildung einer festen Dispersion; und

d) optional, Mischen der erhaltenen festen Dispersion der Schritte b) oder c) mit einem oder mehreren pharmazeutisch akzeptablen Hilfsstoffen.

**5.** Verfahren zur Herstellung einer pharmazeutischen Zusammensetzung, wobei Dapagliflozin in der pharmazeutischen Zusammensetzung nur als amorphes Dapagliflozin vorliegt, umfassend:

a) Bereitstellen einer Lösung von Dapagliflozin der Formel 1

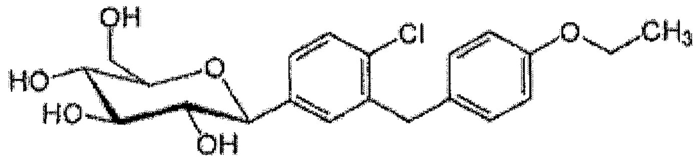

Formel 1

und optional mindestens ein geeignetes Polymer in einem geeigneten Lösungsmittel oder Mischung von Lösungsmitteln;

b) optional, Sprühen oder Dispergieren der Lösung von Schritt (a) auf Trägerpartikel, um Granulen zu bilden;

c) Evaporieren des Lösungsmittels; und

d) Mischen der erhaltenen Zusammensetzung der Schritte b) oder c) mit einem oder mehreren pharmazeutisch akzeptablen Hilfsstoffen.

**6.** Feste Dispersion oder pharmazeutische Zusammensetzung erhältlich durch das Verfahren gemäß Anspruch 4 oder 5.

**7.** Adsorbat umfassend Dapagliflozin ((2S, 3R, 4R, 5S, 6R)-2-[4-Chlor-3-(4-ethoxybenzyl)phenyl]-6-(hydroxymethyl)-tetrahydro-2H-pyran-3,4,5-triol) der Formel 1, adsorbiert an die Oberfläche eines Substrats

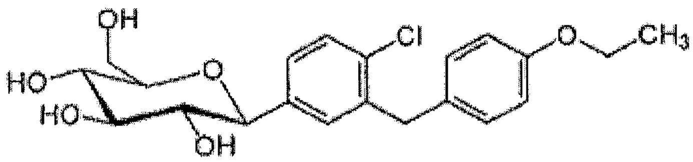

Formel 1

wobei Dapagliflozin im Wesentlichen amorph ist und wobei das Substrat ausgewählt ist aus der Gruppe bestehend aus

(a) einem anorganischen Oxid;
(b) wasserunlöslichem anorganischem Salz;
(c) wasserunlöslichem Polymer; und
(d) einem aktivierten Kohlenstoff,

wobei Dapagliflozin nicht in der Form von Partikeln vorliegt.

**8.** Adsorbat gemäß Anspruch 7, wobei das Substrat ausgewählt ist aus der Gruppe bestehend aus Siliziumdioxid und mikrokristalliner Cellulose.

**9.** Verfahren zur Herstellung eines Adsorbats gemäß Anspruch 7 oder 8, umfassend:

a) Kombinieren einer Lösung von Dapagliflozin der Formel 1

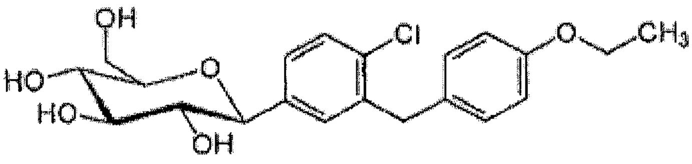

Formel 1

in einem Lösungsmittel oder Mischung von Lösungsmitteln mit einem Substrat;

b) Entfernen des Lösungsmittels oder der Mischung von Lösungsmitteln unter reduziertem Druck, um das Adsorbat zu bilden.

**10.** Verfahren zur Herstellung einer pharmazeutischen Zusammensetzung, umfassend ein Adsorbat wie in Anspruch 7 oder 8 definiert, umfassend:

a) Bereitstellen einer Mischung des Adsorbats, und mindestens eines pharmazeutisch akzeptablen Hilfsstoffs;
b) optional, Fein-Mahlen und/oder Sieben der in Schritt a) erhaltenen Mischung;
c) Formulierung der Mischung von Schritt a) oder b) in eine pharmazeutische Zusammensetzung durch Trockenformulierung.

**11.** Pharmazeutische Zusammensetzung umfassend das Adsorbat wie in Anspruch 7 oder 8 definiert oder erhältlich durch das Verfahren gemäß Anspruch 10, und ein oder mehrere pharmazeutische Hilfsstoffe ausgewählt aus der Gruppe bestehend aus Füllstoffen, Sprengmitteln, Bindemitteln, Gleitmitteln und Tensiden.

**12.** Pharmazeutische Zusammensetzung gemäß irgendeinem der vorhergehenden Ansprüche, wobei die pharmazeutische Zusammensetzung eine komprimierte Darreichungsform ist, bevorzugt eine Tablette, mehr bevorzugt eine Tablette mit sofortiger Wirkstofffreisetzung, und/oder wobei Dapagliflozin in der pharmazeutischen Zusammensetzung nur als amorphes Dapagliflozin vorliegt.

**13.** Pharmazeutische Zusammensetzung gemäß irgendeinem der vorhergehenden Ansprüche, wobei die inhaltliche Einheitlichkeit dieser pharmazeutischen Zusammensetzung, die in einem Packmaterial wie beispielsweise einem Blister enthaltend N Tabletten gepackt ist, angegeben in Bezug auf den Abnahmewert (AV) wie durch die Legende von Tabelle 2 definiert, unter 15 ist, bevorzugt unter 10.

**14.** Pharmazeutische Zusammensetzung gemäß irgendeinem der vorhergehenden Ansprüche, wobei, wenn ein Freisetzungstest durchgeführt wird, bei einem Zeitpunkt von 5 Minuten mehr als 80%, bevorzugt mehr als 85%, des Dapagliflozins freigesetzt ist, wobei der Freisetzungstest ausgeführt wird durch Anwenden der folgenden Parameter: 500 ml Freisetzungsmedium 0,1 M HCl, Apparat 2 bei 50 rpm, Scheitelbehälter, 37°C.

**15.** Feste Dispersion, Adsorbat oder pharmazeutische Zusammensetzung gemäß irgendeinem der vorhergehenden Ansprüche zur Verwendung bei der Behandlung von Krankheiten im Zusammenhang mit Hypoglykämie.

**Revendications**

**1.** Dispersion de solides amorphes d'au moins un polymère approprié et de dapagliflozine ((2S,3R,4R,5S,6R)-2-[4-chloro-3-(4-éthoxybenzyl)phényl]-6-(hydroxyméthyl)-tétrahydro-2H-pyran-3,4,5-triol) de formule 1 :

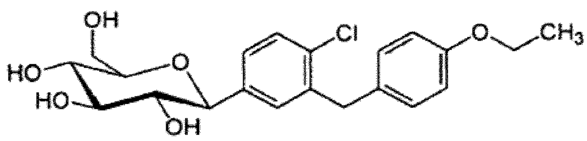

formule 1

dans laquelle le au moins un polymère est choisi dans le groupe constitué de polyvinylpyrrolidone (PVP), d'alcool polyvinylique (PVA), d'acide polyacrylique (PAA), de poly(éthylèneglycol) (PEG), de poly(oxyde d'éthylène) (PEO), d'hydropropylcellulose (HPC), d'hydroxypropylméthylcellulose (HPMC), de copovidone, d'acétate succinate d'hypromellose (AQOAT), de polyacrylates et de leurs mélanges,
et dans laquelle la dapagliflozine est présente sous forme libre.

**2.** Dispersion de solides selon la revendication 1, dans laquelle le rapport pondéral de la dapagliflozine et du au moins un polymère est de 1:10 à 10:1, de préférence de 1:1 à 1:10.

**3.** Composition pharmaceutique comprenant la dispersion de solides selon la revendication 1 ou 2 et un ou plusieurs excipients pharmaceutiquement acceptables choisis dans le groupe constitué de charges, de désintégrants, de liants, de lubrifiants et d'agents tensioactifs, dans laquelle la composition pharmaceutique comprend de la dapagliflozine comme seul ingrédient pharmaceutiquement actif.

**4.** Procédé de préparation de la dispersion de solides selon la revendication 1 ou 2 ou d'une composition pharmaceutique selon la revendication 3, comprenant :

a) la fourniture d'une solution de dapagliflozine et d'au moins un polymère approprié dans un solvant ou un mélange de solvants approprié ;
b) la pulvérisation ou la dispersion éventuelle de la solution de l'étape (a) sur un support pour former des granulés,
c) l'évaporation du solvant, dans lequel l'étape d'évaporation est de préférence réalisée par séchage sur lit fluide, par séchage par pulvérisation, par séchage par congélation (lyophilisation), par séchage sous vide, par séchage en plateau, par séchage à micro-ondes ou d'autres procédés qui entraînent une évaporation du solvant, ce qui entraîne la formation d'une dispersion de solides ; et
d) le mélange éventuel de la dispersion de solides obtenue des étapes b) ou c) avec un ou plusieurs excipients pharmaceutiquement acceptables.

**5.** Procédé de préparation d'une composition pharmaceutique, dans lequel la dapagliflozine est présente dans la composition pharmaceutique uniquement sous la forme de dapagliflozine amorphe, comprenant :

a) la fourniture d'une solution de dapagliflozine de formule 1 :

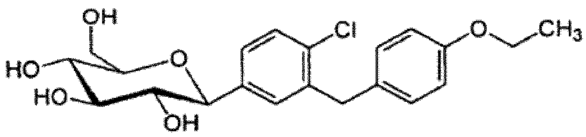

Formule 1

et éventuellement d'au moins un polymère approprié dans un solvant ou un mélange de solvants approprié ;
b) la pulvérisation ou la dispersion éventuelle de la solution de l'étape (a) sur des particules de support afin de former des granulés ;
c) l'évaporation du solvant ; et
d) le mélange de la composition obtenue des étapes b) ou c) avec ou plusieurs excipients pharmaceutiquement acceptables.

**6.** Dispersion de solides ou composition pharmaceutique qui peut être obtenue par le procédé selon la revendication 4 ou 5.

**7.** Adsorbat comprenant de la dapagliflozine ((2S,3R,4R,5S,6R)-2-[4-chloro-3-(4-éthoxybenzyl)phényl]-6-(hydroxyméthyl)tétrahydro-2H-pyran-3,4,5-triol) de formule 1 adsorbée sur la surface d'un substrat :

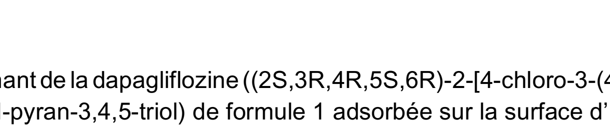

Formule 1

où la dapagliflozine est sensiblement amorphe et où le substrat est choisi dans le groupe constitué des suivants :

(a) un oxyde inorganique ;
(b) un sel inorganique insoluble dans l'eau ;
(c) un polymère insoluble dans l'eau ; et
(d) un carbone activé,

dans lequel la dapagliflozine n'est pas présente sous la forme de particules.

8. Adsorbat selon la revendication 7, dans lequel le substrat est choisi dans le groupe constitué du dioxyde de silicium et de la cellulose microcristalline.

9. Procédé de préparation d'un adsorbat selon la revendication 7 ou 8, comprenant :

a) la combinaison d'une solution de dapagliflozine de formule 1 :

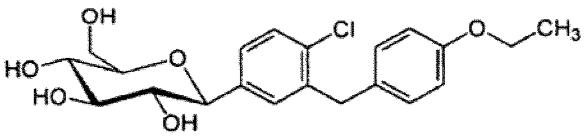

Formule 1

dans un solvant ou un mélange de solvants avec un substrat ;
b) l'élimination du solvant ou du mélange de solvants sous pression réduite pour former l'adsorbat.

10. Procédé de préparation d'une composition pharmaceutique comprenant un adsorbat selon la revendication 7 ou 8, comprenant :

a) la fourniture d'un mélange de l'adsorbat et d'au moins un excipient pharmaceutiquement acceptable ;
b) le broyage fin et/ou le tamisage éventuel du mélange obtenu à l'étape a) ;
c) la formulation du mélange de l'étape a) ou b) en une composition pharmaceutique par formulation à sec.

11. Composition pharmaceutique comprenant un adsorbat selon la revendication 7 ou 8 et qui peut être obtenue par le procédé selon la revendication 10 et un ou plusieurs excipients pharmaceutiquement acceptables choisis dans le groupe constitué des charges, des désintégrants, des liants, des lubrifiants et des agents tensioactifs.

12. Composition pharmaceutique selon l'une quelconque des revendications précédentes, dans laquelle la composition pharmaceutique est une forme de dosage comprimée, de préférence un comprimé, mieux encore un comprimé à libération immédiate et/ou dans laquelle la dapagliflozine est seulement présente dans la composition pharmaceutique sous la forme de dapagliflozine amorphe.

13. Composition pharmaceutique selon l'une quelconque des revendications précédentes, dans laquelle l'uniformité du contenu, indiquée en termes de valeur d'acceptation (AV) comme défini par la légende du tableau 2, de ladite composition pharmaceutique emballée dans un matériau d'emballage tel qu'un emballage pelliculé contenant N comprimés est en dessous de 15, de préférence en dessous de 10.

14. Composition pharmaceutique selon l'une quelconque des revendications précédentes, dans laquelle, lorsque l'on effectue un test de dissolution, au bout de 5 minutes, plus de 80 %, de préférence plus de 85 % de la dapagliflozine sont dissous, dans laquelle le test de dissolution est effectué en appliquant les paramètres suivants : 500 mL d'agent de dissolution 0,1 M de HCl, appareil 2 à 50 tr/min, récipient max, 37 °C.

15. Dispersion de solides, adsorbat ou composition pharmaceutique selon l'une quelconque des revendications précédentes pour utilisation dans le traitement de maladies en rapport avec l'hypoglycémie.

**Fig. 1**

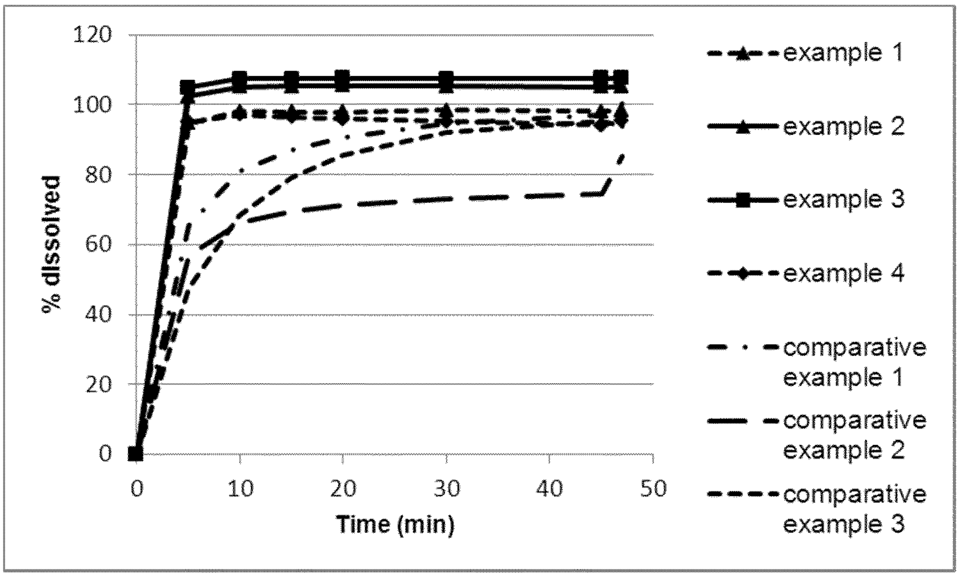
120
100
80
60
40
20
0
% dissolved
0
10
20
30
40
50
Time (min)
example 1
example 2
example 3
example 4
comparative example 1
comparative example 2
comparative example 3

**Fig. 2**

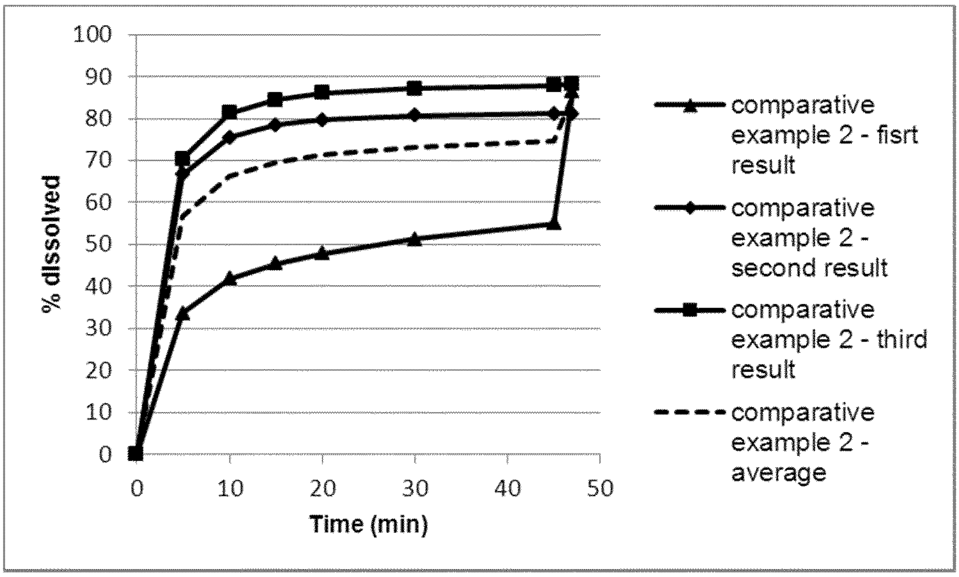
100
90
80
70
60
50
40
30
20
10
0
% dissolved
0
10
20
30
40
50
Time (min)
comparative example 2 - fisrt result
comparative example 2 - second result
comparative example 2 - third result
comparative example 2 - average

Fig. 3

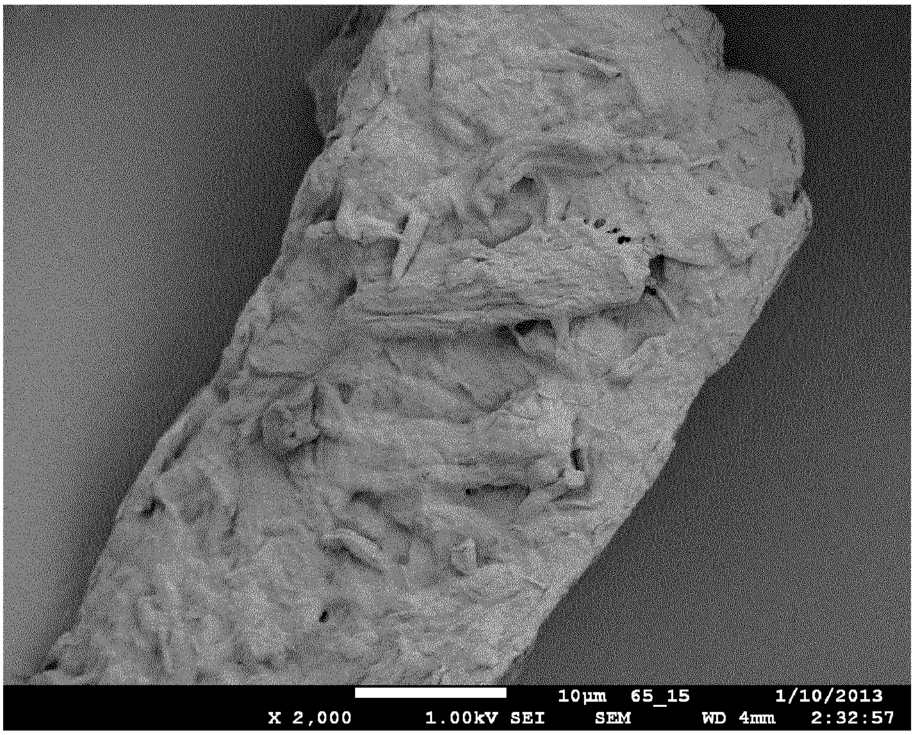
10µm 65_15 1/10/2013
X 2,000 1.00kV SEI SEM WD 4mm 2:32:57

Fig. 4

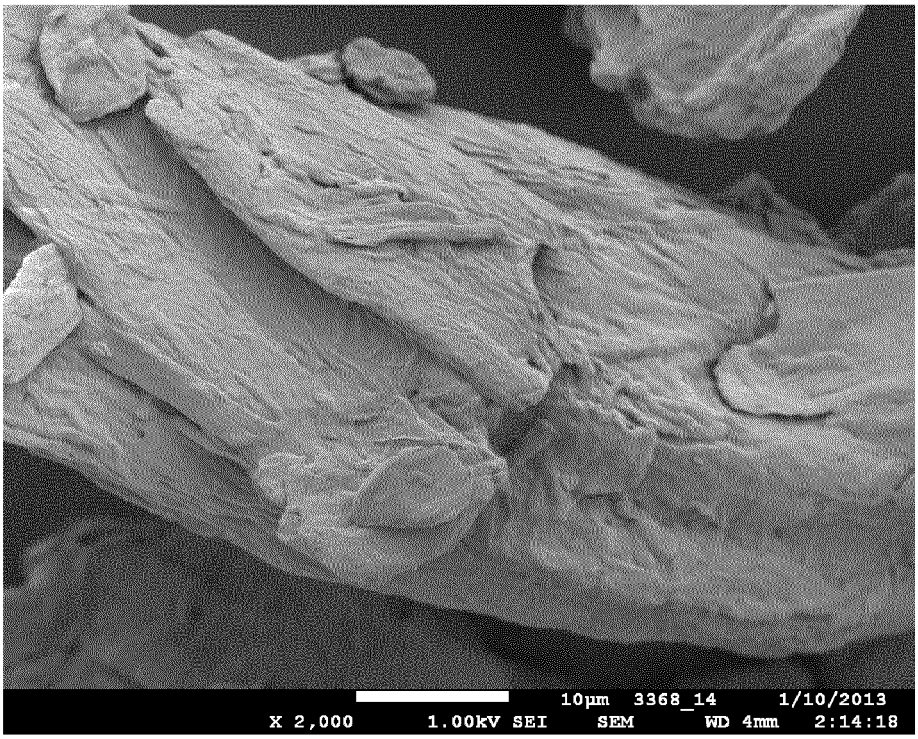
10µm 3368_14 1/10/2013
X 2,000 1.00kV SEI SEM WD 4mm 2:14:18

Fig. 5

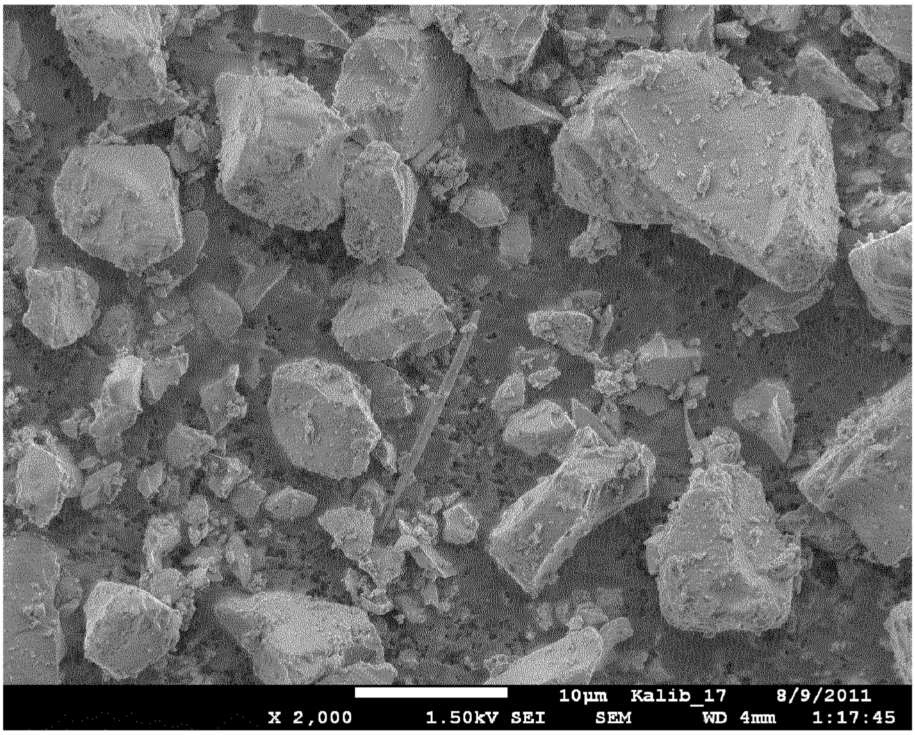
10µm Kalib_17 8/9/2011
X 2,000 1.50kV SEI SEM WD 4mm 1:17:45

Fig. 6

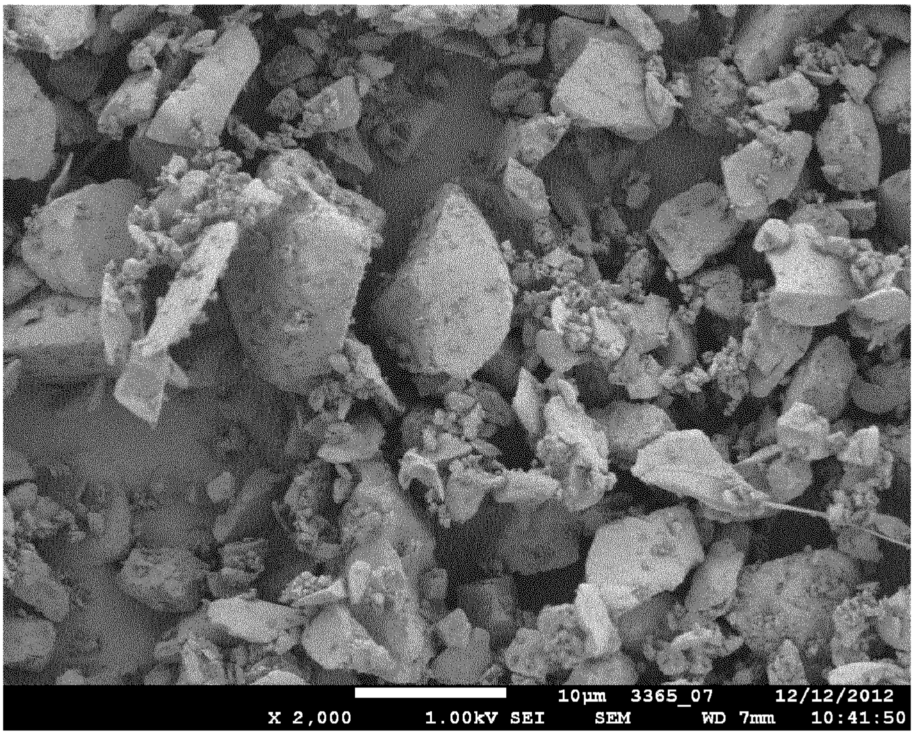
10µm 3365_07 12/12/2012
X 2,000 1.00kV SEI SEM WD 7mm 10:41:50

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03099836 A1
- WO 2008002824 A
- WO 2008116179 A
- WO 2012163546 A
- WO 2013079501 A
- WO 2011060256 A
- WO 2011060290 A
- WO 03000238 A
- WO 2009080698 A
- US 20120088774 A
- WO 2010115886 A
- WO 2004063209 A


### Non-patent literature cited in the description


- *J. Am. Chem. Soc.,* 1938, vol. 60, 309
- **COSMETICS ; RELATED AREAS.** Fiedler Encyclopedia of Excipients for Pharmaceuticals. Wissenschaftliche Verlagsgesellschaft Stuttgart, 2013
- **BAUER ; FRÖMMING ; FÜHRER.** Lehrbuch der Pharmazeutischen Technologie. Wissenschaftliche Verlagsgesellschaft Stuttgart, 2012
- **AUGSBURGER ; STEPHEN.** Pharmaceutical Dosage Forms: Tablets. Informa Healthcare, 2008, vol. 2